# EUROPEAN PATENT SPECIFICATION

(11) **EP 1 198 598 B1**
(45) Date of publication and mention of the grant of the patent: **06.12.2006**
(21) Application number: 00953015.5
(22) Date of filing: 05.07.2000
(51) Int. Cl.: C12Q 1/70

(54) **PROPHAGE DNA SEQUENCES AND THEIR USE IN DETERMINING INSTABILITY IN LACTOBACILLI CULTURES**
PROPHAGEN DNA SEQUENZEN UND DEREN VERWENDUNG FÜR DIE BESTIMMUNG DER INSTABILITÄT VON LACTOBACILLI KULTUREN
SEQUENCES D'ADN DE PROPHAGES ET UTILISATION DE CES DERNIERES POUR DETERMINER L'INSTABILITE DANS DES CULTURES DE LACTOBACILLES

(30) Priority: 08.07.1999 EP 99113282
(43) Date of publication of application: 24.04.2002
(73) Proprietor: SOCIETE DES PRODUITS NESTLE S.A., 1800 Vevey (CH)
(72) Inventor: BRUESSOW, Harald, CH-1814 La Tour de Peilz (CH); DESIERE, Frank, CH-1006 Lausanne (CH); PRIDMORE, Raymond David, CH-1000 Lausanne 26 (CH)
(74) Representative: Chautard, Cécile
(86) International application number: PCT/EP2000/006351
(87) International publication number: WO 2001/004359

(56) References cited:
- DATABASE EMBL [Online] ID/AC: X13616, 19 March 1999 (1999-03-19) BARASH ET AL.: "ENTEROCOCCUS FAECALIS GENOME CONTIG. SEQ. ID NO: 679" XP002127572
- DATABASE EMBL [Online] ID/AC: L03428, 5 October 1992 (1992-10-05) KURAMITSU: "STREPTOCOCCUS MUTANS G PROTEIN GENE SEQUENCE" XP002127573
- DATABASE EMBL [Online] ID/AC: AF109874, June 1999 (1999-06) MCGRATH: "BACTERIOPHAGE TUC2009" XP002127574
- KODAIRA ET AL.: "GENOME STRUCTURE OF THE LACTOBACILLUS TEMPERATE PHAGE PSI-g1e: THE WHOLE GENOME SEQUENCE AND THE PUTATIVE PROMOTER/REPRESSOR SYSTEM" GENE, vol. 187, 1997, pages 45-53, XP002127566 cited in the application
- CHANDRY ET AL.: "ANALYSIS OF THE DNA SEQUENCE, GENE EXPRESSION, ORIGIN OF REPLICATION AND MODULAR STRUCTURE OF THE LACTOCOCCUS LACTIS LYTIC BACTERIOPHAGE SK1" MOL.MICROBIOL., vol. 26, no. 1, 1997, pages 49-64, XP000866448 cited in the application
- HENRICH ET AL.: "PRIMARY STRUCTURE AND FUNCTIONAL ANALYSIS OF THE LYSIS GENES OF LACTOBACILLUS GASSERI BACTERIOPHAGE PSI-ADH" J.BAC., vol. 177, no. 3, 1995, pages 723-732, XP002127567
- VASALA ET AL.: "GENETIC AND BIOCHEMICAL CHARACTERIZATION OF THE LACTOBACILLUS DELBRUECKII SUSP. LACTIS BACTERIOPHAGE LL-H LYSIN" APPLIED AND ENVIROMENTAL MICROBIOLOGY, vol. 61, no. 11, 1995, pages 4004-4011, XP002127568 cited in the application
- LUCCHINI SACHA ET AL: "The structural gene module in Streptococcus thermophilus bacteriophage variant phiSfi11 shows a hierarchy of relatedness to siphoviridae from a wide range of bacterial hosts." VIROLOGY, vol. 246, no. 1, 20 June 1998 (1998-06-20), pages 63-73, XP002154730 ISSN: 0042-6822
- KROGH ET AL.: "THE PHAGE-LIKE ELEMENT PBSX AND PART OF THE SKIN ELEMENT, WHICH ARE RESIDENT AT DIFFERENT LOCATIONS ON THE BACILLUS CHROMOSOME ARE HIGHLY HOMOLOGOUS" MICROBIOLOGY, vol. 142, 1996, pages 2031-2040, XP000857879 cited in the application
- HENDRIX ET AL.: "EVOLUTIONARY RELATIONSHIPS AMONG DIVERSE BACTERIOPHAGES AND PROPHAGES: ALL THE WORLD'S A PHAGE" PNAS, vol. 96, March 1999 (1999-03), pages 2192-2197, XP002127570
- BRÜSSOW ET AL.: "MOLECULAR ECOLOGY AND EVOLUTION OF STREPTOCOCCUS THERMOPHILUS BACTERIOPHAGES-A REVIEW" VIRUS GENES, vol. 16, no. 1, 1998, pages 95-109, XP000867201
- DESIERE FRANK ET AL: "Comparative genomics of the late gene cluster from Lactobacillus phages." VIROLOGY, vol. 275, no. 2, 30 September 2000 (2000-09-30), pages 294-305, XP002155242 ISSN: 0042-6822
- ALTERMANN ET AL.: "PRIMARY STRUCTURE AND FEATURES OF THE GENOME OF THE LACTOBACILLUS GASSERI TEMPERATE BACTERIOPHAGE PSI-ADH" GENE, vol. 236, August 1999 (1999-08), pages 333-346, XP002127571

### SEQUENCE LISTING

<110> Société des Produits Nestlé S.A.
<120> Prophage DNA sequences and their use in determining instability in lactobacilli cultures
<130> 80196
<140>
   <141>
<150> 99113282.0
   <151> 1999-07-08
<160> 5
<170> PatentIn Ver. 2.1
<210> 1
   <211> 35605
   <212> DNA
   <213> Lactobacillus johnsonii
<400> 1
   ctagcatagc tgcagctgta tcagcatttg catctttaat tagcattatt ataagttacc 60
   attggaatag aaaaacatat aaggcaaatg ttgaaattga gccaaaatta gaagcattat 120
   atactttaag aaaattaatt ccagattata ttgctgaaat aaattatgtt acttatttat 180
   actgtaaggc tgcagctaat caaaatgatg aaagacgagc taaggagaat atacttccag 240
   acggtgtaat ttggggtaat attacttttg aagatcacga tagacaaatg gcaaaaacca 300
   agctcgttca cgaacattta acagctattc ttagattaga aggggctgca ttactgttaa 360
   aggatgctca agagctttgg aattgtctaa gtttaaggaa agaatattat aaagaagcta 420
   caaatgaatt cgtatctaaa aaagaaaaag aattcaatca tttgttaaat gaaacaagta 480
   ataaattaaa taatgatttt atagaatatt ataaaagtaa aattgaactt tatgaaaaag 540
   gaaagtcagc ataagctggc tttttatttt acattaaatt gaaaaaaggt ggtgtggtga 600
   tatgtcatga gcaaaataga agatgcaaaa gcagattact tagcaggaat gaagtacaaa 660
   gatattgcta agaaatatga agttgcatta agtactgtta agtcatggaa gactagaaat 720
   aagtggcaac gtaataatgc aaccaaaagg aaaagtatgc atacaaaaca aaaaagtacg 780
   cgtacaaaac gagaaaaggt tgcaccatca ttaccatcac cagaattacc aaataatgat 840
   gaacttactg ataagcaaag agccttttgc ctgtactatt tgcaacgata taatgcgacc 900
   tgggcttatc aaaaagctta tggcggaaac tatgaaactg cattgcgagc aggaccaaga 960
   ttgttgggaa atgttggagt taaaaattac ttagctgagc taaagaaaca acaatcgcaa 1020
   gatctatatg cgaccgcaaa tgacatattg ctgcgctatt taaagcaggc gactagtgat 1080
   gttaccgatg ttctgtcttt taaaacgaaa aagcgcttgg tttattacaa aattcatgat 1140
   aaagatggtc catacgagga tgctggaggt aagttcaggt atgagcctaa gattgatcca 1200
   gagacaggtg aacaagcgta ttactatgaa catttaatta ctctgaaaga tagtgaagat 1260
   attgatacgt caaatattaa gagtattcga atagataagg gtgagcctgt agttgaaatg 1320
   gaagataggc aaaaggcgat gcagatttta cttgatcgct tgcctgaacc agaagttagt 1380
   gatgacagta ctacttcctt gttacgtgct cttaaaaatg gtatggataa gatttggagt 1440
   aaagataatg aagataacta gatttaactt tgttccgttt tctcgtaagc aactgcaagt 1500
   cttaagctgg tggagtaatc ctcaaattct aaaccaggaa gctattattt gtgatggttc 1560
   agttcgtgct ggaaagactg tagtaatggc gctctcatat atattgtggt ccatgactaa 1620
   tttttcaggg caacaatttg gaatggcggg aaagacgatc ggatctttta gacgcaatgt 1680
   acttcgccca ttaagaagta tgttagaaag cgaagggtat aacgtatatg attctcgctc 1740
   tgagaatatg ataactatca gcaaaaatgg gcatacaaac ttttacttca tctttggtgg 1800
   taaagatgaa gcatcacagg acctggttca aggtattact ttggctgggt tcttttttga 1860
   tgaagttgcc cttatgccgc aatcatttgt taatcaagca acagctcgtt gttcagtgac 1920
   aggctctaaa atgtggttca actgtaatcc gtctggcccg tttcattggt ttaaactaaa 1980
   ttggattgac cagatgaaag ataagcgtgc tttacgcatc cacttcacga tgcatgataa 2040
   tccatccctt gacagtgtaa ctattaacag atacgagcgt atgtattcag gagtgtttta 2100
   tcagagatat atacaaggtc tatgggtaat gtcggaagga gttatctatg ataattttga 2160
   taaagacaca atggtagtta atgaattacc caatcatttt gaaaagtatt atgtatcttg 2220
   tgactacggt acattaaacc ctactgcgtt cctattatgg ggacgcaatc atggagtttg 2280
   gtacttggtt aaggaatatt actactcagg aagaactaca tcccgtcaaa agacagatga 2340
   agagtattgc catgatctca aagagtttct tggagatatt cgannnnnnn nnnnnnnnnn 2400
   nnnnnnnnnn tgaaggaaaa ataaagttag catgaactgt cctaatctgt taaggaatta 2460
   gctagttatg tttggatgat aaagcagcag aacatggtga agataaacct gttaaacagc 2520
   atgaccatgc ttgtgatgca atgagatatt tcgtatacac aattatttac aagaaagtta 2580
   ctgcaaaagt tactgtacga cctagagtac gtggcttata gaaggaaggt gtaaatgtgg 2640
   cagttgtaat tgataaagat ttacttgacg atgtgaatga acccaatatt gaagcaatta 2700
   actatgcgat tagagaattg caaaatcgca aaaaaaggtt agacaaactg tctgattatt 2760
   ataatggtaa gcaagaaatt gaaaagcatg agtttgataa tgctaccgtt gaagcagcta 2820
   atgtgatggt taatcatgct aaatacatta ctgatatgaa cgtgggcttt atgactggta 2880
   accccgtgaa atatgttgct gaaaagggga agaatattga tgatattcta gaagttttta 2940
   atcaaattga tatccataaa cacgatattg aactagagaa agatctgtcg gtatttgggt 3000
   atggatatga gttgctttac cttaaaaaaa ctgatccaat atctgttaga gatgaactag 3060
   ggaacgagaa acttactcca aatactgaat taaaaattga agtaattgac ccacgggcaa 3120
   ctgttgtggt ttgtgatgat acagttgagc atgatccttt atttgctgta tttacacaag 3180
   aaaagaagga cttagagggt aatactaatg gttatagcat tactgtttac atgcctcagc 3240
   gaattgttga atatcgcaca aaaacaacta tggaagtttc agcaaatgat ccaattgttt 3300
   atgatggaga gaatttattt ggtgcagttc caataattga attcagaaac aacgaagaaa 3360
   gacagggaga ttttgaacag cttatctctt tgattgatgc ttacaaccta ctacaaacag 3420
   accgaatttc tgacaaagaa gcgtttgttg atgcattgct ggttacattc ggatttggcc 3480
   taggggatga caaggatgat attcaacgat taaaacgtgg cgcaatcgaa gctcctcctc 3540
   gtgaagaggg agcggatatc gaatggttaa ccaagagttt tgacgaaact caagtcaact 3600
   tacttagtca atctattgaa aatgacatcc acaagatttc atatgtgcca aatatgaatg 3660
   atgagaagtt catgggcaat gtttctggtg aagcaatgaa atttaagttg tttggtttag 3720
   aaaacttgtt atcaattaaa cagcgatact tttttgatgg cttacgtcga agattgaaac 3780
   taatccaaac tattgtaaac atcaagggtg ctaatgatga tgctagtgga tgtaagatct 3840
   cacttgtagc taatattcct tctaatttat ctgatgtagt taataacgtt aaaaatgcgg 3900
   atggaataat tcctagaaaa tatacttata gttggcttcc tgatgttgat aatcctcaag 3960
   atgtcattga tgaaatgaat caacaagatg ctgaaacaat taaaaagaat caagaagcat 4020
   tacgtgggca agatccagat cgtttagaat tggaggataa gcaagatgat tcgagtgaga 4080
   atgataaaga agccggatca aaccataatc agagccaccg gacacgcgca gtatagtgtt 4140
   aaaggatctg atattgtttg tgcttcattt tctacgctta ttactcatac tgtaaataat 4200
   tgtacgaaag taactgcaac tgataaggat ggcgtattaa ttgctacatt ttcagatcct 4260
   aaaagtattg aaaataagac gctattaaat gcatttgaaa atacagttaa ccaattaatt 4320
   gaccaatacg ggcaatacat ttgttggtgt tgatggtcta tgaaagttaa taagaagaaa 4380
   tttacgtatt ggcagttacg tgatttgcaa gatgagcaga gaaatcaaga tgaagcaacc 4440
   gaaaggttaa aaataattaa taatgcatat caaaaagcac aatcatattt aagcgacgag 4500
   gttcaaaaaa tctatcgtcg ctatttttat gcagatattt caaaagatga agttgcaact 4560
   atcatgtcat cgcacatttc gccttctgaa ttggttactc ttaaagcttt atcgtctagt 4620
   atcacggaca aggaaagtag gcaagcagtt gataattact taagtagact tgcagctaag 4680
   agtagaatta cacgacttga agaaatgcaa cttaaagcat acattgctgc aaagtcagct 4740
   ggtgcaactg aattagatca aaatgttaaa ttgcatactg acataatgaa acgtgcttgg 4800
   tcagaggcag aaaaacaaag tgctgtttat gacaccacta aggattacac attacactct 4860
   cctcactctg tagaagttaa acaggataaa atagtcatta aaaatcctga tactggtaaa 4920
   gaagtggcta ctgttccgat ggataaggat gttccaaaga gtaaaatcac tgaaatacct 4980
   aatcgctatg ttgaaaaatc attagaaact cgctggaaag gaaagaactt ttcagcgcgt 5040
   atctggggaa atactgataa attagctgaa cgactgcagg aattgtttac ggttaaagaa 5100
   ctgagtaatc tccctgaacg tgaaatgatt aagcgaattg aacaagaatt taatgttggt 5160
   aagttttatg ccagtcgctt gattaggact gaagctaatt tcttttattc taaaattaag 5220
   ctagataatt ggcgtaaacg tggagttaaa caataccaat tgctagcagt cattgatagt 5280
   cgtactagta agatatgtag atcaataaat gggaatattt ataatgttaa agatgcagta 5340
   tttggtaaga atgttccacc attgcatccc ttttgtcgga ctgttcctgt aatttatctg 5400
   ggtaatgcta gaagtgctaa taataaaccg gtaaaaaagt aattcgtaga cctaagcaag 5460
   tcataaaact gcattgaaaa ctaaatatac gtatgtggat ttgttactgc atatcttgaa 5520
   gtcaccgtgt agaaatatgg ggtggctttt ttcatgcagt taatttgcaa atgttcatgg 5580
   gtagaaagga ataattatgg aaaataaatt ttatgaaggt ttattaaaac ttgatttaca 5640
   acgatttgct gatgaaggac aaggtgaggg aggtgatgac agtaataatg cgcaagataa 5700
   tgtaaaagat gatagttcaa cagatgaaca gccttttaaa acatttagtt ctcaatcaga 5760
   actggattct tttgtagata aaaaattagc taaggcttta gatacggctc gctctaattg 5820
   ggaaaaagaa caaaacgata aggcacaaaa agccaaagat ttaaaggaaa tgtccccaga 5880
   agaacgacaa gaatatgatt taaagcaacg tgaaaaagct ttaactgatc gtgaagccga 5940
   agtaactaag cgtgaaaata agagtaaatt agccacacaa ttaattacag atggtttgcc 6000
   ggcagaacta gtggatgttt ttgacgatgt gctagctgat gaggataaga tgaccaatac 6060
   ttatcaaaag gttagtgatg tttttcgaaa tgctgttcat gacgcagttg aaactcgact 6120
   agctcaaagt gcaagacctc caaagagctt aggtgatact caaactaaaa agtctactgg 6180
   agaaatgttt gctgaaagag ctaatgaaac tcaaaaggta aaaaatgatt tttggaatta 6240
   gaaaggaata gatatgtata cacaatttca aaatggcaag cagctaaact ttttagcttc 6300
   tgaaaagttc actgctttcc cagaaacaat taataaagat aattacaacg tccaaacaga 6360
   tgacttagga cgtaaatatg taccagcggg aacggtatat ccaactaatg atgcaaaagc 6420
   tgtcggaatc actgtaaatg atgtgtacgt gtcagaagat ggatctaatc aaatggttgc 6480
   tgttatgcgt gatggttggg tgttaagtca aagattgaat ccaactccaa cagcagaggc 6540
   tattaaggca atgtcagcaa ttcactttaa agatttggat acaacgggag ttgcatctgt 6600
   tagtacccta aaggactaat agaagggaga atataattaa tgaaaaatca aaaactacaa 6660
   ttagatttac aacgatttgc aacacctatt cttgatatgt ttagtcaaaa tacggtgctt 6720
   gactataccc gcaatcgtca atatccagaa atgttaggtg atactttatt cccagcggtc 6780
   aaagtaccaa cacttgaagt tgatattttg aaagctggta gtcgtgtacc aacaatcgca 6840
   agtgtttcag cttttgatgc tgaagctgaa attggcactc gtgaagcaag taagatgact 6900
   gctgaattgg catacgtgaa gcgcaaaatg caaattactg aagaaatgct aatcaaattg 6960
   caatcaccac gtaatactgc agaagaaaac tacttaaaac agtatgtttt tgatgatatt 7020
   gacgctatgg ttcaagcagt taaggcacgt ggcgaaaaaa tgactatgga aatgtttgct 7080
   actggtaaaa ttactgataa gaaaaatggc attgctattg attatggcgt gccaaagaaa 7140
   catcaagaga cattatcagg taccaagacg tgggataaga gtgatgcttc tattattgat 7200
   aacttgcaag attggtcaga ttcactagat gttacaccaa cccgagcttt aacttctaag 7260
   aaagttttac gtatattaat gcgtagtact gaaattaaag aagctatctt tggtaaagat 7320
   actggacgtg ttgttggcca agctgattta gatcaatgga tgacagccca agggttacca 7380
   attattcgtg cttatgatgg taagtatcga gatgaagatt caaggggcaa tttaacaact 7440
   aactcttact tcccagagga tcgcatcgtt cttttcaatg atgaagtacc tggacaaaag 7500
   atctatggtc caactccaga agaaaatcgc ttaatttcaa gcaatgctca agtatctaat 7560
   gttggcaata ttatggccaa gatttatgaa actagtgaag atccaattgg aacatggatt 7620
   ttagcgtccg caaccatgct tccatcattt gctagtgcgg atgatgtttt ccaagctaag 7680
   gttctttaat taagggaggt gcttaatgtg gatcaactaa cggaaatagt ttcagcttta 7740
   agtactcgat tagaaaatgt taataatgcc ttactaactg aactagttaa agaatcgata 7800
   gcacaggtac tagattacac tggtcaaaag aaattagttg gtagtatgga tatttacgtt 7860
   aaaaaactag cagttattaa ttacaatcgg ttaggcattg aaggcgaaac acaacgttcc 7920
   gaaggcggta taactaacta tcttgagact ggcattccaa aagatattcg acaaggatta 7980
   aatagttatc gaattgctaa ggtgaaaaag ctatgagatt aaaagacagt gatcttataa 8040
   ctgtttatct tagagaacca atgaatactc aagatgatga aggttatagt atttgtggct 8100
   ggggtgatcc acaaccaatt agaatgaatg ttcaatcagc tggtggtacg gtcaatgctc 8160
   aactttatgg gaaagatatc aagtacatca aaacatgtaa gtatcaaggt gatttacttg 8220
   cagagggaca tggtgaaggt tttggcgttt gtctaaaggt ttccagttct agtgatcctg 8280
   attacaagat tacggctatt caggagtttt ctactcataa aaacgttact ttagaacgta 8340
   taaagaggga tgaacaaaat gattgaagtt gaacttaaag gagtcaatga attaagagca 8400
   aagctaaaga aattaccgga tataatggca aaagcaacag ctaatgctca agaaaatgca 8460
   attgagcagg cggaagccta tgcagttgat gagttgcaat ccagtattaa atactccacc 8520
   ggcgaactta ctcgaagctt taaacatgaa gtgaaggttg atggagatga agttattggt 8580
   cgttggtgga ctcttctatg gttgctgtat ttcgtgaatt tgggactggg ttagttggtg 8640
   aacggtcaca taagcaactt cctaagaatg ttgctattat ttatagacag acgccatggt 8700
   tcttcccagt tgattcagtt gatcttgatc tcacaaaaat atatggcatt cctaagatta 8760
   agatcaatgg aaaatatttt tatagaacta ctggacagcc agcaaggcaa tttatgactc 8820
   ctgctgctaa taaaatggct aaggaagcac cagagattat aaaaagatca attgaccaag 8880
   aacttcatga taaattgggt ggttaaatgg aaatctataa tgttaaagca ctagtgttta 8940
   agactttaaa gtctatgccg gaattaaaac tagtttcacc atcttatcca gataaattta 9000
   caacattccc agcagctatt tattcaacta gtcaatcttc ttatatacgt aatgcacaac 9060
   aagaagagac agacactgaa tggaagataa caattgattt atataatgac catggatcat 9120
   taactaatat aaaagcaaag ctcattgcta gattttcagc aatgggcttt tctaatagtg 9180
   ttggtgacca agatttaaat ggagtatcac gtgtggtaat agttttcgca ggaattgtag 9240
   ataatactag ccatcgtgta tatcagaaag gataaaaatg aacaacgtaa aattttatag 9300
   tgatttatta acattagacc ttcaacgttt tgcagttgat agctcagaag gtcttgcagg 9360
   aacaggaaca acactcgaaa gatcagtaga tggatcatct tgggatgaaa ttgctgatat 9420
   taaaaccatc cctgaattgg gtggagacac tgaaaaaatc gatgtaacta ctttggcaga 9480
   tgatagacgt aagcaaattg aaggtattca aaatgcttct aacgttcaat tccaagcagt 9540
   atataagggt gctagttttg caaaagctct ggcgcaggct ggtgatagaa agcaatacca 9600
   atggaaagtt acatatccag atggaatgac agctacgatg aaaggatcat acaacattaa 9660
   atttggcgca gtatcagtta acggcgcatt aggttacaca atcactatta ctgtatctga 9720
   tggacctaca tttgctgctg caggagcagc tcattaaatt ggtaagtaat agaaagtgat 9780
   aggcgtgggt tcgattccta cgtctatttt tatacaacat gaataggaga aattatggca 9840
   acaacaatta agaaagcaac taaaacagta cagctaggtg atcttgaact taatttaaaa 9900
   ctaggtggcc gtgagatttt taaaattgaa cgtcgtctag gtaaatcaat gctttcactt 9960
   tttatggatt cacaaggcgg aaataaatta cctcctgtaa atgaaattct tatcgtatta 10020
   caaggagcta atcaaaatca cggtgtaaca gataaacgag ttattgaagc ctttgaaaag 10080
   tatttagacg atggtcatac cactatggat ttgttcaatg aattaatgga attatttgat 10140
   gaatctggtt ttttcggcaa gaaaaagaag aaggctacaa agaccaatat ggaatcggac 10200
   gaagtaactc tagatccagt ggaagcagaa agcgaattga tgtagatgca aatcatttcg 10260
   agacagtatc tgatctattt agacatctgt atccaatcgc agtggaatca ggtatagagg 10320
   ctgaccattt ttgggatatg gatttcggtg aaattatgac ccaaatttca gcaaatgaaa 10380
   aacgtcgatt ggaagattta cgagctaaag cttatatgga tcatcgccta agtgaaatga 10440
   tggcttttgc ttttaacgat ccagcaaaaa tgcccaaagt tgaagaagct tatccatttg 10500
   tgaaagataa tttggatcaa acaaatgaga ggtctgagga agaacccaat tggaaaaaag 10560
   atcagattat ttttatgcag caagcccaga aaatcaaaca atttaataaa gataaaggag 10620
   gtggaaatta atggatctag aagaacttga gttaagattt agagcaaatt atggggatgt 10680
   cattcaaaag attgatgaat taactaatct tattgtgcaa aaaacaggtg atatgcaata 10740
   taagatgcaa aataatttag ataaatttca gcaatcaatg aatgagagta cttcaagagc 10800
   taatgaaaat gttaaagaag agattcatca acgtagtgaa gcggaagaag ctaaacaaaa 10860
   attacttgag cagactctta atactcaaaa tgaggtaaca gataagattg ttcaaggaaa 10920
   taaagaacaa gccgaaagct ctaaagaagc tgttaatcag tcagaaaaga gcctggatag 10980
   tttaacagct cgcttgcaag aagcttctaa tatgcaacaa cgaattgcac aacaaactag 11040
   tacagctcat gatgtaataa aagatatttc tagacccaaa tcacaaacgc aagtaaaaga 11100
   accattaaag tccacttcac agaatgacta ctcaagcttt gataactatc aagaaaaacg 11160
   aattcagagt tatatgccaa agcgtcctgt tgatttaggt attgatgatg aaatacaagc 11220
   tgaagcatcc agagctaaaa aagaagttga tagcttagta actcacatca acgagaagat 11280
   ggaacaggct cgatcaatgc aacggagaat tgctacttta acagctaatc gagacaatct 11340
   ggatatgagc aagcagggta gtaaagttaa agcaatgaga ttagatgatc aaattgstga 11400
   cgctcaaatt aagatggaac ggtatcagac tcaagctaag gctcttgcac aggaaatgtc 11460
   gcaggagctt gatactgttc ctaactcatt aagacgcatt gagcgtgaaa tggatcaaac 11520
   ggaaggcaag attgaacgaa ttagacgttc tattgctgaa atgagggata atgatgcgac 11580
   tcttggccga tcctcgggca ctaataaaga gattaagaat gctgaagctg agtacagaag 11640
   attagttgct agaagtaatg agttagcaaa ggcatataca tacgtcagtg cacgtggaga 11700
   tgagttaaga aatagttctt cacgaattaa tactgaatta gctgaagaaa acagaaatgt 11760
   ctcaaatctt ggttctaagt ttagcaggct aaaaaatact atttctaatg ttaattcatc 11820
   tcttagacga tttggtaata gtgggaattc ttctatacgg aaagctggat caggcgtttc 11880
   agttttaagt gaacgactaa ggggcgttcg aatggcaatt cgaatgttag ccagtcaatt 11940
   aattgtgttt actctgctct atcaaggaat tatgatgctt gcccaaggta tgggtgcagc 12000
   attaatgact aatcgacaat ttgcaagtag ttttaacgca attaaggtta acttgttgac 12060
   tgctttttat ccaatttata gttatgtttt acctgctata aatgctttta tgagttcatt 12120
   gcaaaaagca actgcatgga ttgctcaatt tacttctgcg ttaacaggaa tgagcttgtc 12180
   tagtgctaga agtggtgctc atggtttgta cgatcaagta caggcaatga atgatacttc 12240
   aaaggctgca agtaaagcta atgaagctgt taaaaaacaa cagcaagaac aagctaaagc 12300
   agttcaacgt gctaatcagc aaattgctca agctaatcgt caaggagcgg cagccgttgc 12360
   agcagaaaat gaaagaataa aagcatctaa cgaacaagct aaaaaagctt ttgaagatac 12420
   aaagaaagct aatgaagacc ttcaagcatc actcatgggc tttgatgagc tgaacgtttt 12480
   agataacagc aaaaacaata acgataatgg tagttttgaa gctcaaccac tagaaaaatt 12540
   tactccacag caaaagcagg atacacctat ttttgatgat cccggaatag atgatggtga 12600
   gacaggtgga aatcaggggc ttgattggaa tgttccatta gttgcatccc aaaatgctat 12660
   tgatgctgct aataaagtta aaaaggtttt aggcgaaatc tttgatccaa tgaaaaaagc 12720
   ttgggatgaa aaaggccaag ctgttgttga tgcagctaag tattcatgga ctgaaattag 12780
   gcgcttatta ggggatgtag gtaattcttt cttacatgtt tgggacaatg gaacaggaca 12840
   aaaagtaata gaaaatctac ttcaactttt agccgatatg ctgaacgtca ttggcgacat 12900
   ttcacgagct tttgcagaag catgggaaga gagtggtcga gggaccaagt ttattcagac 12960
   tatcttcaat tctctcaata atgttcttga aactatccat catatagctc aagcgtttcg 13020
   tgatgcatgg aatactggtg atctaggcaa gagaattttt gctaatttgc ttgatttagc 13080
   tactaagtta gttgggtttg ttggcgatat tgctaaatcc tttgatgagg catggcaaca 13140
   tggtaatgcc ggaactagac tatggcaagc ttggctaaat gcactgaata atatacttga 13200
   tacttacaag catatagtta catcaattga tgaagcatgg aaacactcca acttaggcgt 13260
   ttcaatttgg agtcatatta ttcaaatagt aactggtgtg ggaaacacta ttggcaattt 13320
   agctggtcaa tttgataaag cgtggcaaca tggaaatgtt ggaacttcta tctttaaaac 13380
   tttgctcggt atggtagatg acatgcttgg atcacttggt gatatggcaa cgtatactgc 13440
   aaactgggct aagaagcttg attttacccc attgcttcag tcaattgata atctgctaaa 13500
   agctattcga ccagtaacca aagatgtttg ggatggatta tcgtgggcat acaagaatgt 13560
   cttgcttcca ctcgcacgtt ttacaattac taaggtaatc cctgagttct taaatgcctt 13620
   agctgctgct cttaaagtcg tacatagtgt aatcaaagcg gcagaacccg ttctagaatg 13680
   gttcttcgat agttttatta aaccattggc taaaatagct ggttttgcaa ttgtagaggc 13740
   tttaaaactt ctaactaaag cacttgaggg cttatctgat tggatagatc atcatcaaac 13800
   ggccgttaag atcatgactg caacgttgct tacgctttta ggtattaagg ttgctaaagc 13860
   tactattgct ggaattcaaa gctttactga tactctgaag atccttgcaa tgctcaaatt 13920
   tgataaattg aaagctgctg caaagtatgc tgatgattta ttaggagtag caattgaatt 13980
   tgcaaagcac ccaataacta atattaaaga acttgcaaaa ttaagttttg aaaatatcaa 14040
   gggtggctat aaccgcatca aagatctttg ggtgaagtaa aataaaggct ggcaggatag 14100
   taatcttgcc aagaccgatt ttcttaaatc tgctcgctct tcaatacaat ctggcgaacc 14160
   aatgaagctg gggcaaaaac ttggtactgg cttatctggt gctatgattg ctgtaacttc 14220
   aggaattgat atctacaaag gaatcaaagc taataacaaa gaagaaaaat ttgcagattt 14280
   tggttctgga attggtggtg cagttggtgg tgccattgga ctctggttcg gtggcccact 14340
   aggggcagcc gtaggacaac agattggctc attaattggt aaatggggcg gtgttggtgc 14400
   ctctaaattt ggtgatggtt ggtctaagta tggtaaaggt aagaaaccta aagactgggt 14460
   tgaagccata ggctttaagg cccatgaaat cttagataac tttacttctt gggctaaatc 14520
   cgttggtaga gatattaata acaatatttc taaaggtaaa aaagatgttc aaactgctag 14580
   ctctaatatc cataaatggt ccactaattt tatttctggt gcaaagaaag atattaaatc 14640
   ttgggcacaa aatatcggtt ctaacattca taaagatgta gacaaaggta agaaactagc 14700
   taaacaagct ggagataaag taaaagaatg gtctactagc tttattagtg atgctaaaaa 14760
   gaaagttcat gattggtctt ctcaaattgg ttctaatgta aataatagcg ttgaaaatgg 14820
   tcaagccatg gctaagaatg ctggaactaa aattaaaaat tggactaccg actttagaga 14880
   atcagccagt ggactagtaa gatcatgggc cgaaagacta ggcagaacac attaataatg 14940
   gctctgaatc atctcgctct ggagccacta atgccggtaa taaaatttct caatggacaa 15000
   gaagtttctt tggaggagcc aaccaaagtg tttctagttg ggccagtggt ctaggcggtc 15060
   atgttaatga cggaataggt ggagcttata actctgctaa aaatgctggt gaacgtctag 15120
   gaagctgggt ttcaagcttt agacatgata cttcaagaaa acttggttca tgggctggca 15180
   ctcttgggag cacaattggg aatggaatta ctgatggact gcacaacatt agtcgagccg 15240
   ttcgaaaagt ggttaatgca attgttaaac cggtgcaaaa tgctactgat caagttagaa 15300
   aaggtattaa ctgggtactt ggtaaacttg gtggtgggtc aattggctgg ggattctttg 15360
   attggaattc ttatgcgaat gggacgaata gccacccagg tggactagcc ttggttaatg 15420
   atcaagccgg agacacttat cgggaaagtt atgaattacc taacggagaa caaggtttat 15480
   tcccagctga acgtaatttg ttaacttact tgccagctgg cacaaaagtt aaaactgcaa 15540
   ctgatacggc taatgaatta gcaggtatgg ttcctaaata tgctggtggt ataggtaact 15600
   ttaatttcga ttttagtggt atctttagcg gaattagtag cgctcttgga aacctaaatt 15660
   ttgggaatat atttgatggg gttggcaatt ttgttgatgg tgtaatggaa gaacttgaaa 15720
   aagttactga tgatatagct catccagaac gattagttaa ctacattatt gataaatttg 15780
   tcacctatga ttggagttta ggcgacgctt cattaaagtt tgctaaaggc gctgttcatc 15840
   aagaaaagaa aggcatgatg aattgggcta agaaagttat caatcaattt ggtggtgcaa 15900
   ctcatcaaac agggctgggt gcggaaggtt ggcgtagtgc tgttaagaaa gcattgcgca 15960
   agaatggtct acctgcaaca ccagcatatg taaatgcatg ggttcgtcaa atccaaactg 16020
   agtctggtgg taatgaacat gctgtacaag gcggttatac ggatatcaac accttaacag 16080
   gagacttggc aaaaggattg ttgcaaacta tctctgcaac ttttaacgct tataaattcc 16140
   ctggtcacgg taatatcttt aatggttacg acaacatgct tgcagcaatt aactatgcta 16200
   agcatcgcta cggttcagac atgcttgctg ttatcggtca cggacatggc tatgaagacg 16260
   gtggcttaat tgctaaacac ggtttctatg agattagcga aaacgacaag ccagaaatgg 16320
   ttgttccttt aactaatcgt gagttaggta tgcgaagaat taatgaagct attgcattta 16380
   tgaatcagaa ttttggtggt ggcttgcaaa tgccatcttc cttaagcaga cgaactgcta 16440
   ttgatagttc aatttattct gatactcaat ctaatgactc tacgtcagtt cagcgtggag 16500
   gattcaaaga gatgagtaca gaattagtaa atgctatagt tcaagcaatt caaatgcaaa 16560
   actttaatag taataacgga aaacctattg atttacactt atctgtcaag attggtgatg 16620
   agtcatttgg cgaacacgct attaaaggta ttaatactat caatcaaaag aatggtagaa 16680
   acatgttaaa tctttagaag gaggaaatga gtgattgtat tctttaaaaa tttctgggac 16740
   agtggttaat cctgccccgc aaactatgca ggtaacaatt caagatattg acgcaaaagc 16800
   aacgcgtgat gcgcaaggat tattgcaccg agatcgagta gctactaaaa gaaaaattac 16860
   attggcattt ggtgcactca cggttccgga atgtgcaaag attttaggag cagttaaaag 16920
   tgaatttttt atagtagatt acttagatcc acaagatgga aatatgcggt caggaacatt 16980
   ctatgtggga gatcgaacaa cacctgttta ttcctttgta gattcggtcc ctgtttggaa 17040
   aggcttatcc tttgatttga tagaacagta ggaggtgaga aatattgcta acacaaacga 17100
   aagaagttag ggatgcttgg cgagcatcac aacgaacgtt agatattaaa gtggcagtta 17160
   atgggaaaac ttataccgct accgatatca acagtttgaa atatgattca ggagcttata 17220
   ctggtgacac gtttgccatt ggctcaacat actcaaatag tgttcaaatt gaattttcac 17280
   atttaataga gaatctaaaa cttggtatgg aagttttacc aagtatcggg attaagacgt 17340
   ctagtggcta tgtatatgag ccgttgggcg tttttattat ctctagtgaa atcaagatgg 17400
   atcgcaataa caatcttact tccattagtg caagtgatag attttgcggg ttagaaggat 17460
   cctataaatc taagctggct tatccagcaa aagttttaga tgtaattgct gaaatctgtg 17520
   aacaatcagg agtcaaggct aatgtggatg atctggctag acttccacat caagctgact 17580
   tacctagacc tatcactggt caaacatata gaaaggcttt aggttggatt gcacaactat 17640
   atgccgggta tgctacgttc gatcgtaaag gtttatttac gattagaact atcgcagagc 17700
   ctaattatga gttagatccc agtcagtatg aacaagctgg tttaactaaa aatgaagctc 17760
   catatagaat tagcggtatt cagtgtcaaa cgacaattac cactaaaact agggatggtg 17820
   aagatactga tgaaactaag aattatcaag ttggagatac gaatggatct caaattaagc 17880
   ttgaaaacaa tattatgacg cctgataggc tgactaatat ttgggagcag attaaagatg 17940
   ttaatttcta tccatttagt ttgaattggt ttggaaatcc tgcaattgaa gcgggagatt 18000
   ggttaaaact acaagacaag caaggcaata agtttattgt tcccaacaat agctacacac 18060
   ttgattttaa tggaggactt tcagcaactt ctaaggcaga tcaaacttct tccacagact 18120
   ctgtaatagc ttgggaggga actttctctc aaactattag agaacttcaa ggtcgtaaag 18180
   caccagatgg aacagtgatt tttccgccta gtgtaactga accacctacg aatgctaaac 18240
   ctaacgatgt ttggttcaag caaaatggta attcaacaga attgtgggtg tttactgaac 18300
   aagaagatgg gactagaaaa tggattagga gggatttaac tcctgacgag attaagaaac 18360
   aagttcaaga agcacaagat ggcttgaaag atgctaagaa agaaatagca gataatcttg 18420
   ctaaagcgga taaagatatt gctgaactta atgaaagtat taacaatcaa aaaggttcac 18480
   ttgatggtct aagtactaca gtcaataccg ttgttattcc taaagtaacc gatgtaacta 18540
   accaagtttc tgatgctatt aaaaaagtaa atgaacagaa aaatattgtt actggactgc 18600
   aaaatcaggc tattcaacaa ggaaaagata tctctaagat tacttctgat gttcatggtg 18660
   taactgttga tctagctaat ctcaatgggg atgtaaacca gacaaaagcc acagttcaag 18720
   gccttcaatc aacgttagca atgcccaagg tgatattgcc cagatcaaag tagatgcgaa 18780
   gaaattggaa actagtctat ctggtaaagt tgataattct acttatgcaa cttttgttaa 18840
   tcaaactaat caagcattaa atgctaagtt gattgctagt gatctgaatg gatatgctaa 18900
   aactactgat ttgcaagcta cggctaatgg attgcagttt aatatcaata gcgtaacgga 18960
   taaattgaat aatctmagaa ttggaggtag aaatttagtt ggtgggactg acaaagaata 19020
   cgtaatggga tttggaattc ctaataccgt ttggaaagat aattttgctt atatctcttt 19080
   accgttaatt agtggtgatg gaggcgaaat tttaccccaa ggtgcaggat tttggcatgt 19140
   attaagtcca ggagaagaat atactcaaac catttggatc gaaacagatg ctatcataaa 19200
   aagtttaaat ggtacatttt taacatggct aactatggac gatgggcatg atgtgcaaaa 19260
   agcatatatt caaaggatag gcaataacag ctataaaatt gtaggttctt atacttggcc 19320
   acaaaataaa aaaggcaata gagttcgctt atttgatatt tttagtttgc caaattcgat 19380
   tgacttgaaa agtggaacat atctaaagtt tggaaaacta aagttagaag tgggaaatgc 19440
   ttcaactgac tggactccgg ctgttgaaga tactgaacac gatctagaaa gtttgtctgc 19500
   acgcataact attaatagtc aacaatttgg ttcttactat actaaaggtg agtctgacaa 19560
   tagaactaat actgcaaaaa atgaagttat caattcaatt aagaatgata gtaattggca 19620
   tggattaacc aatatactaa cgaatagtgg ctttatacaa accgcagatg gctttataca 19680
   aaaagtacag caaacgatgc aaccaattat taatgaaaat aatagtggtg gagtgaattt 19740
   attaactaat acatatactt tgcgtaactt taataatgcc ggtaatgctg gagaagctac 19800
   tggatttgaa attgattctg gagatagcca taaaaacgaa ttaaatgctg gtgaaataag 19860
   cacaaatgtt tttcatgttt atgccaaaga tttaaatcat gctcctgttt actttggtca 19920
   aaactttaca ctacccaaag gaacttggac tctaagtttt ttagttcgtc ataatggaag 19980
   tagtgaacaa aaaatcagac taagctttta tacagacgat tgtgctaaca gaggatggat 20040
   tcctctagga acatctgatg agatagataa catttggaaa agacatcaga taacttttac 20100
   aacaacccag gaaatttttg aacaaaatcc gagagtacat atgccaactg atgatatagt 20160
   ccccggtggc tctttgtatt ttgcaaattt taaattagaa tccggatcaa ttgctactac 20220
   ttggtgtcct gctcccgaag atttagctaa gcaagtagca gttactgaat taggtcaaac 20280
   cataaaaggg cttcaatcaa ctgtttcaag taactacgga aatctacaga gccagataag 20340
   tcaaactgct ggaactatta ggaatgaaat aactgataga accaataatc tacaaagtca 20400
   aattactcaa aatgctaata atattagtat taaagtaaat gccgtggggg atttatctaa 20460
   catatgttta aatccaacat ttattgatgg aagtactgaa ggttgggaaa acgtatttag 20520
   ttcgtctgga gatcccggta gtcctacaaa gttttacggg ggagttaata caagaaatgc 20580
   gttttacgga aatatgttct cagtggcggc aggggataaa tattttttct cggtctttgc 20640
   ttggcaaaat caatctacta atcctttaaa tataggattt acttatttgc aaaaagatgg 20700
   aacttggaat tggcaatccg ctataaattt tgctcctaat gaaggtgcgc gaactaaatc 20760
   aggatctatc actattccta aggaagcagt taaagccaga atatgggtag aaattgattc 20820
   attttctaat tttggaaatt ggtggtttac caatcttata gttagaaaga atgatactat 20880
   ctcgcaaatt aatatgtcag ctggcacaac tttaatccag aatgacaaaa tatatatgga 20940
   tgcaagctca accgtatttt ctggtaaagc cttcattcct gatgcagcaa taactaatat 21000
   atcagctgat aaaattaaca ctggtacact cgatgccggc agaattaatg taattaactt 21060
   aaatgcaaac aacatcacta ctgggacaat taagggacaa aatagtagtt ttaatttaac 21120
   tgatggaacg ttaacggctt taaatcagta taatgagggc gtttttatga gaaatgggaa 21180
   acttgagttt acctcacgtc aaagctggaa taataactcc acagctacat atggatatat 21240
   tcaatcttta cccaaaatgt ttgccttagg atataactat ggtggattag atattaatgg 21300
   tactagtggg tttgtactgc ataatgataa aacgaaaatt aagactagta atagtggaca 21360
   gagtggttca gcagttgaag gaggaagcta tatttttgga tctgatggtc ttactagcat 21420
   aaataatgaa aattcagtga ttattaatgc aggtgctcaa tcatacagtg agtggacggc 21480
   tggaataagc ttattatcag ggaatgcatc tagaaatgtt cctccaagta tcactttagg 21540
   atcctctaac agtctcaatg atcgctctac agttactata tctggaagtc ttgcggtatt 21600
   aggatcgaag aatgccgctg ttcgaacatc tcaaggatta agagctatta acgcatatga 21660
   aacagccgaa tattactttg gagatatagg aagggcaaag actaacagta atggggctgt 21720
   ttgcatatac atggatccgt tatttcttga aacagtcaat acaggagttc catatcatat 21780
   atttcttact agttatagtg aggcaagaat ttgggtttca gagatgtatc catcatattt 21840
   tgtaattaga tcagataagc caaatgctga ctttgtttgg gaaattaaag caaagcgtaa 21900
   aggatatgaa aatgatagat taaaaattat agatcaggag aaaaaagtaa atgaataaca 21960
   ataatgcagt atcacaaaaa ttaattaata aactagcggt tagtgagtat aacaatgcag 22020
   ttttagaagc aaaagttgat gagctaaatc aagaagtaaa tcaattaaag gcagaaaagg 22080
   agaataaaga tgttaaaaac aagtaaatca atcgcaattt caggtcgttc aatggtagat 22140
   gataagcaag tagcaacttt taacgctaat atttatgaag ctaatgcttc aggtggtagt 22200
   gacaacatta atatgattat tactgatcgg gatttatatg atgcaaacaa agttactgta 22260
   cggaaggatt tacaagactt tcaaacaaaa gtatggtcag cacaagatga agtgatggca 22320
   ggaagtgatg aaaaggcaag tgaaggttaa tgaatgagat agcaaaacta tgggaaaata 22380
   taaaacaaaa tcctctgcat ttaattattg ggctaatgat ttttttatca ggaagctttt 22440
   tgttactttg tgatgatttc tttctttggc cgcctgaatg gaccaatgtt tttaataatg 22500
   atctgattga cgctattgcg attgtgatag gcgtctttta ttttgcttac gttttagcag 22560
   gtgctaatag tccactagct aacatttgtt tattaacggc tagtgctttc cttttaacaa 22620
   ttctcattgt tttagaaatt gggtgcgttt tagcatttca aaattatcca ttagtaatgg 22680
   cagcaattta tcagttcggt gcattgctat tagttcaagt atgtgcatca agatcaccag 22740
   gcttaaaaag aaagtaggga taagaggtgc aagaatggat tgactttgta caaaaaattg 22800
   catttcttat ttctagcatt gtaggacttt taacaatttt caatactctt aagcaaacag 22860
   actattcaaa tgctaaggaa aagaaggaag agctaaagga agatgtagag ctttatagaa 22920
   aaagatggct tcaggcagaa gaagcctacg ataaactttt gaaagacaat gaaaggctta 22980
   aaagacaagt tactagatta gaaagtagga aaaataatga ataatatttt agttaatgta 23040
   tccgctgtaa ttgttgcatt ggtattgtca tactttgtat tttggtatcg aaatcataaa 23100
   attgaaattg ataaaaaaag agctaaggga gatgctttag cttttgtggt tgatactttg 23160
   ggtcaagttg ctaccaatgt agtttacgat ttaaaggata attcagaaaa aggtactgaa 23220
   aagaaagaac aagctaaagt taaaattaag aatttttatg ctgacgcaca tttaccagca 23280
   cctagtgatg cccaagtttc tggtgcaatt gaaaaggctg tagctataat gaagatgtca 23340
   agcgaaggtg aaaagaatga ctaaagtaga aaaaagaagt tatggagtag atatatccag 23400
   ctttcagggg actgatttat tagctatggg cagatcaggt gctaagtttg ctattgtaaa 23460
   gacttctgaa ggactggact atagaaatcc aaaagcccaa gctcaaatta atagcgctag 23520
   aaacaatgga atgatggtta gtggatatca ctatgcaaga tttagcaata atgctaatgt 23580
   agcagctcaa gaaggaaact atgcagttag ttcagctaaa caagttggca ttgaaccagg 23640
   tagttaccta gcttgtgatt atgagcaagg cagtggaaat gaaactggag gtaatcgtga 23700
   agaaaacacg actgcgattc tagcatttct aaatacaatc gttagtgcag gatataagcc 23760
   tttattatat tcaggtgcag aacttatgaa aaataaggtt aataccgcta aaattttggc 23820
   aaaatatcct aattgcttat gggttgccgc atatccttat aaaggagcaa caaatactgc 23880
   tgatttcaat tatttccctt ctatggaagg cgtgatcatt tggcagttta cagataattg 23940
   gcgtggtatg aatgttgatg gaaacatcaa cttattagaa ttagaaacta aaaaatctgt 24000
   gtcacaacca cgtaacgcac ctggagaatc tactagttgg attgataatt ttggagataa 24060
   atgggttaaa gaggatggca cttttatcac tgatcgacca attaatttga gaaaaggagc 24120
   caagctcact agtgacatta tcactacctt accagctggt tcagtaatta aatatgatgc 24180
   ttatagtaga catggtggtt atgtctggat tagacagccc cgtgaaaatg gccaatacgg 24240
   ttacttagtt tgtcgtcaag gaaatacccg tggggaacat ttaaataaag aaaaaagcca 24300
   ctctggagaa attaattctc tggagtggct tttttgcata caacaaattt tctattattt 24360
   taactatgtt ttcttattac aattttactt acaaaatgtt atacttaact tgcaacaagt 24420
   tagaaattct aaaattcaat gcactcaaaa agcattacct gatcgtcaca aaaggtaatg 24480
   cttttttatc gcttagtgca accttctaag cgttgagggt ggagtaaggc gactattaat 24540
   gctttttgtt gtgatgccga tcatccaacc agtgatcgaa tagtgatttc actatttcca 24600
   caagaatggg agctacaact aaagttagaa attccttcaa tgaactcacc tcctttcaag 24660
   aggaggcaat agtcgctgaa ataattatag caaaatcaaa gtcggaaaag tgattgaaaa 24720
   aagcaagtaa attagctgaa ctttttaaag attttggtta tgaagcttac tggaaagaaa 24780
   aacacgaact tgactttaga gacgaggatc tattagcagt aatcgattta aaagacgtta 24840
   gtcaactaaa agagttagct cttgaaggta aaatttacgt agactttgaa aatcaaataa 24900
   ttgtacccac tagctatgaa gtagatgact aaaaatataa gaggttgttt tagaaaatta 24960
   atttcctagg atgttttgtt tgtggtaaca aatggtaaca attttggtaa catagcattg 25020
   aatagttata tcaacataaa gcaatgaagt ttgatagatc aataattcaa gtggctaatg 25080
   taaacataaa taatcaagca ctgaaacctg tcatcccgat tgaatattat tttgttaaaa 25140
   agttcgtctt gggttagaaa gacgaacttt tttgtatata atgttcatga aatatcaaaa 25200
   agtttacatt tactaattgt aagtgataag atatttgtct aaagattgta atattgatct 25260
   taaaagaagt aaggcggtgc aaaatggaaa aatcatatag tgatagtgca aaaaatgttc 25320
   ttgaaattgc taaagagcaa gcacaaaact ttcaccatcg aattattgga acagagcatg 25380
   ttctgcttgc tttagtaatt gaagctaatg gggatgctgg aaaacttttg cgtgaaagaa 25440
   atgtaacact aaccttagtg cgagaagaaa ttgaaagata tactggctat ggttcaagtc 25500
   caaaggccac atatatggaa atgtccccac gtttaagttt agttttgaat tttgctaaac 25560
   aaagggctga cgaattaggg actgctcaaa ttgagacaaa acatattttg ctaggtttgt 25620
   tagctagtga tcaaatttta gcaagtttaa ttttgaaaaa tattggtgta gatccacgag 25680
   atttaagtca ggatgtaaat gatagtttca tggatggttc tggggaagaa aataatgttc 25740
   ttggtatttc ttcagccact ggtatgaaaa aaagggtaaa gtcattgact cccaaaattt 25800
   tagataaaag tttagtgtga atctaaaata aaagagcccg tgaaggtgca attgacccgg 25860
   taattgggag agataaagaa ataaaaagag taattcaaat cttatctcga agaaccaaaa 25920
   acaatcccgt tttggtagga gaacctggtg ttggtaagac tgcagttgcc caaggaatag 25980
   cagccgcaat tgtaaatcat gaagttccag atactctagc taaaaaacga gttatggcct 26040
   tggacatggg aagtttaatt gctggaacta aatatcgtgg tgaatttgaa gatagaatga 26100
   agaagattct aaaagaaatt caacaggatg gttcagttat cttatttgtt gatgaaatgc 26160
   atactttgat tggtgcaggt ggcgcggaag gtgcaattga tgcttcaaat atcttaaagc 26220
   cttcattagc acgtggcgat attcaaatga ttggcgctac tacttttgat gagtaccaaa 26280
   aatatattga aaaagatcaa gctttagcaa gacgttttca acaagtaaaa attggtgaac 26340
   cttcaaaaga agaaactgtg gatattttaa aacgttgcgc cctaaatatg aaaaatttca 26400
   caatgtaaaa attgaagatg aggcaattaa tgatgcagtt gaattttcaa cacggtatat 26460
   tgctaaccga ttcttacctg ataaagcaat tgatttgatt gatgaagcaa gcgcagctgt 26520
   aaaaattcaa gcaatcggta aaagtgatcc tcgtctaact aaattggata cacaaattaa 26580
   tgatgttatt ccatcagaaa gaacaagctg cagacaatca aaattttgtt caagcagcta 26640
   agttacggga tgaggaaaat aagcttacac aggatcggga taaattaatc gaaaaagtag 26700
   agaataaaaa ctctaaaaag tcgattgttg attctgataa gatcgctcaa attgtatctg 26760
   aatggactgg tgtgcccgtt actagaatga agaagagtga aactaaacgt ttagctcatt 26820
   tagaaagtat tttgcatgaa cgtgtaattg gacaggacga agctatcagt gccgtaagtc 26880
   gagcaattag acgaagtaga agtggaatca aggatgaaaa tcgtccaatc ggttcgttct 26940
   tattcctagg acctactggt gttggtaaga cagaattagc taaggctctt gctgcagcag 27000
   tttttggctc agaacgaaat attattcgtg tagacatgtc tgaatacatg gatcagattg 27060
   caacaagcaa attaattggt tcggcaccag gatatgtagg atatgaagag ggtggtcagc 27120
   tctcagaacg tgtaagacgt aatccatatt cagtaatttt attggatgaa gtagaaaaag 27180
   ctcatccgga cgtgtttaat ttattgcttc aagttttgga tgaaggattt ttaactgatt 27240
   ctaaaggtag aaaagtcgat tttagaaata ccattattat catgacttct aaccttgggt 27300
   cacgtagttt gcaagaagat aagacagttg ggtttgcagc ggataatgct gataaaaata 27360
   aacttcaaca agaaaaagtt acggcggctg ttaaacaatt cttcagacca gaattttaaa 27420
   cagaattgat gagaccgtgg tctttgatag cttaactaag aaacaattgc gtgaaattgt 27480
   tagcttaatg actggtcatt tggtagatcg cttagctaaa aaagacgtaa ccttaaagat 27540
   ttcaccagcg gctttagatg tattggctaa agatggattt gatccagaga tgggcgctcg 27600
   tccattacgt cgtgcaattc aacatgaact tgaagatgta attgctgaag atttaattag 27660
   tgaaaaaatt aaggctgacc aaattgtaaa agttggagct catcaaggta aattaaaatt 27720
   tacaattctt gataaagata acactttagt gaaaaattag ttttttgaca aattagtaat 27780
   ataagtgtaa aatactaaac gtttaaaagg ctgaaattca ggatttcgct gatctattgt 27840
   ccagcgagat gataaaacaa aaacgacatt tacgttgttt ttgttttttt gtacccaaat 27900
   tttgactttt tgcaatttgt aacacaaatg taatatttag attcttatat aagaaaggat 27960
   gttttctttg ttaggacacg ctgtgaacta cggtagtcac cggacaagac gtagcttctc 28020
   tcgaattaaa gaagtattga agttacctaa cttgactgat gtacaaacgc aatcttacaa 28080
   gtggtttctt aatgaaggta ttcgtgaaat gtttgacgac attatgccga tttcagactt 28140
   ttcaggaaaa ctttctttag agtttgttga ttacaagctc ttgaagccaa aatacactct 28200
   tgaagaagca cgtgatcacg atgctaatta ctctgctcct cttcatgtaa ctttgaagtt 28260
   aaccaaccat gagaccggag aaattaagac tcaagacgtc ttctttggag aattcccatt 28320
   aatgactgat tcaggaactt tcgttattaa tggtgctgag agagttattg tttctcagct 28380
   tgttagatct cctggtgttt actaccattc agattttgat aaaaatggtc gccaaatttt 28440
   cggtgctaca gttattccta accgtggtgc atggcttgaa tatgaaactg atgctaaaga 28500
   tttagcttat gttcgtatcg atcgtactcg taagcttcct ttaactgttt taattcgtgc 28560
   tttaggattt ggttctgata gtgaaattgc ggatatgttt ggtgaaagtg attcaattcg 28620
   tttcacttta gaaaaagata tccacaagaa tcctgctgat tctcgcgttg cagaagcatt 28680
   aaaggatatt tacgaaagat tacgtccagg tgaacctaag acaactgatt catctcgttc 28740
   acttttatat gctagattct ttgatccaag aagatatgat ttagctccag ttcggtcgyt 28800
   ataagatcaa taagaaactt tctttaaaga atcgtttack aagacaaact ttggctgaaa 28860
   ctttagctga tcctgatact ggtgaaatca ttgctaaaaa gggtgacgtt gttacccacg 28920
   aacttcttga taaattatca ccttacttag atcgtgatga ctttaagatg gtacttatga 28980
   acttcaaagg aaggtgtgtt acctgatcca gtaacagttc aagaaattaa ggtatattct 29040
   aaagttgatc ctgaacgtgt agttaagtta atgtctaacg gccacattgc agatgatgtt 29100
   aaacatttaa ctcctgctga tgtattggca tcaattaact acttcttcga ccttcaagat 29160
   aatattggta ctactgatga tattgaccac ttaggtaacc gtcgtattcg tcgtgtaggt 29220
   gaattacttc aaaaccaatt tagaattggt ttagcaagaa tggaacgtgt tgtacgtgaa 29280
   agaatgtcaa tccaagacat ttctactgtt acaccacaac aattaatcaa tattcgtcct 29340
   gttgttgctt cagttaagga attctttggt tcatcacaat tatcacaatt tatggatcaa 29400
   aacaacccac ttggtgagtt aactcacaag cgtcgtatgt cagccttagg acctggtggt 29460
   ttgtctcgtg accgtgctgg atatgaagtt cgtgacgtgc actatactca ctatggtcgt 29520
   ctatgtccta ttgaaactcc tgaaggacct aacattggtt tgattaactc tttagcaact 29580
   tacgctattg ttaacaagta tggtttcatt gaaacaccat atcgtcgtgt ttcttgggat 29640
   actcacaagg ttactgataa gatcgactac ttaactgctg atgtagaaga taattacatt 29700
   attgctggts staacgctcg cttaaatgaa gatggttctt tcaaagataa gattgtttta 29760
   gcccgtcata aggaagataa cctagaagtt acccctgata agattgacta catggacgtt 29820
   attcctaagc aagtagtttc agtaacttct gcatgtattc ctttccttga aaacgacgac 29880
   tctaaccgtg ctttgatggg ggctaaccac caacgtcaag ctgttccatt gattaatcca 29940
   catgcaccaa ttgttggtac tggtatggaa taccgtgcag cacatgactc tggggatgca 30000
   ttagttgcta aagctcctgg tgtggttgaa tatgttgatg caaacgaaat tagaattaga 30060
   agagacgatg atactttaga taagtatgtt cttgaaaaat tccgtcgttc aaacgcaact 30120
   aagaactaca accaaactcc agctgttaag caaggtgaac gagtagttgc agatgaagtt 30180
   attgctgatg gtccagcaat ggaaaacggc gaattagctc taggtcaaaa cccaatcatt 30240
   gcattcttga cttggaacat gtacaactat gaagatgccg ttatgatttc tgaacgtatg 30300
   gttaaagatg atgtctacac ttctatccat attgaagatt atgaatcaga agctcgtgat 30360
   actaagttag gacctgaaga aatcacacgt gaaattccaa atgttggtga agatgcactt 30420
   aaagaccttg atgaagaagg tattgttcgc ataggtgctg aagttcaaga tggtgatatc 30480
   ttagttggta aagtaacccc taagggtgtg actgaattat cagctgaaga acgtttgctt 30540
   cacgctattt ttggtgaaaa agctcgtgaa gttcgtgata cttccttgcg tgtacctcac 30600
   ggtggtggcg gtattgttca aaacgttcaa gtattcactc gtgaagcagg cgacgaattg 30660
   ccacctggtg taaataagat ggttcgtgtt tacatcgttc aaaaacgtaa gattcaagtc 30720
   ggtgacaaga tgtctggtcg tcacggaaac aagggtacta ttgccttagt ttgtcctgaa 30780
   gaagatatgc catacttacc agatggtcgt ccagtagata tttgtttgaa cccaatgggt 30840
   gtgccttcac gtatgaacat tggacaggtt cttgaattac acttgggtat tgctgctaaa 30900
   cagcttggta ttcacgttgc tactccagta tttgatggtg cttctgaaga tgatatgtgg 30960
   aatatggttc gtgaagctgg tattggaaaa gatggtaaaa ctgttcttta cgatggacgt 31020
   actggtgaac cgttccacaa ccgtgtttca gtaggtatta tgtactactt gaagttgact 31080
   cacatggttg atgataagat ccacgcacgt tcaattgggc cttactcact tgttactcaa 31140
   caaccacttg gtggtaaagc acaatttggt ggtcagcgtt tcggtgaaat ggaagtttgg 31200
   gctcttgaag cttatggtgc tgcttacaca ctacaagaaa tcttaactta caagtcagat 31260
   gacgttgtag gtcgtgttaa ggcttatgaa gctattgtta agggcgaaag aattccaaaa 31320
   cctggtgttc cagaatcatt ccgcgttctt gttaaagaac tacaatctct tggtttagat 31380
   atcaaggtgt tggatatgga tcataaagaa attgagttac gtgatatgga tgatgactct 31440
   aacgatcatt tcaacatcga tactttatct aaacttgctg aacaacaaga aaagaagaag 31500
   ttagctgaag aagctgcaaa aaaggatgat aagccagatg aacctgtaga tgaaagtgat 31560
   tcttcaactt catctgatga taaggtttct aagtaatata ggaggttaaa cttttgatcg 31620
   acgtaaataa gtttgaaagt atgcaaatcg gtttggcttc tccaaacaag atcagaagtt 31680
   ggtcttatgg tgaagttaaa aaaccagaaa ctatcaacta tcgtactttg aaaccagaaa 31740
   aaagacggtt tgttcgatga aagaatcttc ggaccaacta aggactggtc ttgtgcttgt 31800
   ggtaagtaca agggtattcg ttaccgtggc attgtttgtg atagatgtgg tgttgaagtt 31860
   acttctgcta aagttagaag agaacgtatg ggtcacattg aactagctgc accggtttca 31920
   catatctggt atttcaaagg tatcccatct cgtatgggat tagtcttaga tatttcacct 31980
   cgtgcattgg aagaagtaat ttattttgct gcttacattg ttattgatgc aggtgatact 32040
   gatcttgaag ataaacaact tttaacagaa gctgaatatc gtgaaaagaa agcaaaattc 32100
   ggtgaccgtt ttgaagctaa aatgggtgct gaagctgtaa aagaacttct agaacaagta 32160
   gatattgata aagaagttca tgaactaaaa gaagaattaa agactgctac tggtcaaaag 32220
   agaactcggg ctatcagaag attagacatc ttggatgcct ttaaaaattc aggtaacaag 32280
   ccttcatgga tggttatgga ttgtattcct gttattccac cagacttgag accaatggtt 32340
   caacttgatg gtggacgttt cgcaacttct gacttgaatg atttatacag acgtgtaatc 32400
   aaccgtaaca atcgtttaaa gagattgtta gacttaaatg caccaagaat tatcgttcaa 32460
   aacgaaaagc gtatgcttca agaagcagtt gacgcattaa ttgacaatgg tagacgtgga 32520
   cgtccagttg tcggaccagg taatcgtcct cttaaatctc tttctcacat gttgaaaggt 32580
   aagcaaggac gtttacgtca aaacttactt ggtaaacgtg ttgactactc aggtcgttca 32640
   gttatcgatg tttcacctga attgaagttt taccaatgtg gtgtaccacg tccaatggct 32700
   ctagaattat ttaagccatt tgtaatgcac gaattagtaa aacgtggttt agcatctaac 32760
   attaagaatg ctaagcgtaa gattgatcgt gaagatgatg atatctggga tatcttagaa 32820
   gatgtaatta aagaaagacc agttctttta aaccgtgcac ctactcttca ccgtttaggt 32880
   attcaagcct ttgaaccagt tctggtacct ggtaaatcaa ttcgtcttca cccacttgct 32940
   tgtgaagctt acaatgccga ttttgatggt gaccagatgg ccattcacgt tccattatct 33000
   gatgaagctg tagcagaatc acgtttgctg atgcttgctg ctcaccatat cttggctcct 33060
   aaggatggta agccaatcgt tacaccttct caggatattg ttttgggtaa ctactggtta 33120
   actcaagcag agcgtggtcg tgaaggtgaa ggaatgatct ttgattcacc agctgaagca 33180
   gctattgcat atgcaaatgg tgatattcac tatcatactc gtattggttt agcagctgac 33240
   tcaatgccag aaaagccatg gccaaagggc tacgaacatg gaatttttgt aactacttac 33300
   ggtaagttag tattcaacca aattttccct aaggatttct actacattaa cgatcctact 33360
   caagagaatc ttactcaccc agtagatgaa agatacttct tacaaccagg tgaagatatc 33420
   catgaaaagc ttgacaatat gaaacttggt caagccttta agaagggatt cttatcagat 33480
   tctattgctc aaatttacaa ggattacaaa gttcaaagaa cttctgattt cttggatgat 33540
   ttgaaggaac taggctacac tgtatgtact acttctggtt taactatcgg tgttgaagat 33600
   atccctacaa ttagtgataa agatgatatt gtagcagaag ctcgtaagaa agttgatgtt 33660
   gtttctaagc aataccgtcg tggtttaatc actgatgaag aaagacatga tcgagtaatt 33720
   agtatttgga ataactgtaa agacattgtt caaaatgaaa ttgctcaaat tattcatgca 33780
   ccaagaaacc caattacaat catggccgat tctggtgctc gtggtaacat ttctaacttt 33840
   actcagcttg ctggtatgcg tggtttgatg gccsstccta acggaggaat gatggaaatt 33900
   cctgttactt caaacttccg tgaaggtttg tctgttttgg aaatgttcat gtccactcac 33960
   ggtgctcgta agggtatgac ggatactgcc ttgaagactg ccaactctgg ttacttaact 34020
   cgtcgtttgg tagacgtagc tcaagatgtc attattcgtg aagaagattg tggtactgat 34080
   cgtggtttaa ctgttcacgc aattactgaa ggcgatgaaa tgattgaacc attatttgac 34140
   cgtttagttg gtcgctacac ttctaagagt gtttacgatc cagaaactca tgaagtaatt 34200
   tgtccagctg atgtcttaat ggacgaagat atggctcata agatcgttga tgccggagtt 34260
   actgaagtaa caattcgttc cgtatttact tgcaacactc aacatggtgt atgtaagaaa 34320
   tgttacggta tgaaccttgc tactggtgac gacgttgaag ttggtgaagc agttggtact 34380
   gttgccgctc aatcaattgg tgaacctggt actcagttaa ctatgcgtaa cttccacaac 34440
   ggtggtgttg ccggtgctgc agatattact caaggtttac ctcgtgttca agagttgttt 34500
   gaagctcgta atcctaaggg tcgtgctaca atttctgaag taactggtga agtaacttca 34560
   attgaagaag atccagccga acacactcgt caaattaccg ttaagggaca aactgatact 34620
   cgtacttatg acgtaccata tactgcttca gtagcagttg ctgaaggcga tcatgttgta 34680
   cgtggagata agttgactct tggttctatc gatcctaagg aattgattcg tgtacgtgat 34740
   gcattgacta ccgaaaaata cattctaagt gaaattcaaa aagcttatag aatgcagggt 34800
   gtagaaattg ccgacaaaca cgttgaagtt atggcacgtc aaatgcttca aaaggttcgt 34860
   attcttgacc caggtgaaac cgatatctta ccaggtgaat tgatggatat tggtgaattt 34920
   aaggcaagaa accgtgaagt aattatttcc ggtggtattc ctgctactgc gcaaagtgta 34980
   attcttggta ttaccaaggc cgctcttgaa actaacagtt tcttatctgc agcttccttc 35040
   caagaaacta ctcgtgttct tactgatgct tctattcgtg gtaagaacga tccattactt 35100
   ggtcttaagg aaaatgttat tattggtaag attattcctg caggtactgg tatgccagtt 35160
   taccgtgaaa tggaacctaa agttgatgtt cctgaagatg aaaagaaaaa gtcagtttat 35220
   tctatagctg atattgaaaa gaaattagct gcagctgatg cagagaaaga caatggttca 35280
   gctgactaat cgtcctttca aaacttaaac atttgtgtta aaaaagccta ttcgtgtaga 35340
   aacgagtagg cttttttgtg ttattagaga aaattataaa atataagact taaaaaaata 35400
   aaaggaacaa agggataggt gtttcgtttg tcaccaacta tgtgattaat aagtaaaaga 35460
   atagaagcta aaaggaaaat atattgtgca gtataaaaat catagtaaaa aatcagaata 35520
   ataaaaatga ttgtatcacc atagccgaat ttatttttta aattcattat tagaaaaatt 35580
   aaaattgtag taattattag gatcc                                       35605
<210> 2
   <211> 15561
   <212> DNA
   <213> Lactobacillus johnsonii
<400> 2
   agtcgatatc ctgctachca aacagaagct aaggcgaaag ttttaaatga taagaaagaa 60
   ccattagaca atattcaaat aggcgataag tttgctagta ttggcgactc gcctattttt 120
   tatgccgtga caaagattaa tgctgactta acggttgaac taacagcgga agttaagggt 180
   tccagcgcta atagttatat cgggcagatt ttacctgtta chccaaacga cttactttca 240
   tgggctgaaa ttacagaaat tactgcccct gcaaggatgt agaaagtgac gatcacttaa 300
   gagcaagatt gttaagctct caaagctgga ttgcttacgg tggtaatgta gctgattact 360
   tagacatgac aggcaagatt gatgaggttg gagctgcaca gatttatcca acttggaacg 420
   gtggtggaac tgttaaagtt gttattctga ataataattt aatgcctgct agtgctagtt 480
   tggttcaaaa ggttaaaaat gtacttgatc cagaagacaa gcaagcagag ggctatggat 540
   tagctccaat tgaccatgct gtgactgtaa ctgctcctga aaagttgatt gtaaatgttg 600
   atatttcagt aaagcttgat gacacgaaag taacacggta cgtgaaagat agcattacta 660
   aggcagttga gggctacttc caatcattga gagaagattg ggcagacatt aatcaaaagc 720
   ttggtagagg ttatcaagaa acaatttatc gttctaagat cctgtcccaa gttatgctga 780
   cggaaggtgt ggttaatgct aagcttccat ctctaaatgg tcgtgacgca gacattgact 840
   tagttttcac taattcaaag tcacaattgc cagtggtcgg gacggtgaca attaatgagc 900
   aataagtatg agcttctaaa ctatatgcct gattattacg atggcgtata tgagatggag 960
   gagctcctta aatccgaaag tttaacgctt aaagatttag aagacagcca tttgaggaca 1020
   ttgctaaatg aatttgtctc aactgctgat gctaagggaa tttcactctt tgaaagtcaa 1080
   ctaggcatag ttccagacga aaacgatact ttggaaatgc gcagaaataa ggtcttgatg 1140
   tatgtactac caccaaggcc aataacaatt agatttttca aagatatgct taataacgtt 1200
   aatcttccag tgaaaattga tgttaattat ggagctagag ctgttgtagc aactgctaaa 1260
   tctgctgaaa tgacaagtaa tcagattaat tacttgaagt atctgttgaa tgtttatttg 1320
   cctgctaatc tgctatatca agtaaaaatc ttgctaaata gtgctaaagt ttcaaatgat 1380
   ctaagacttg gcattggtaa cattgtaaaa gcaacaggca tagcaaaagc aagtcctagc 1440
   caagtattta actagtgagg tgatgaaatg tcagaatata ataaaacaat tttaaccaat 1500
   gaaggtatcg acttagcccg cagagctaac aagggtacgg caacattctc tttgacgcga 1560
   ggtgtttcat caactgataa cttatcagga aagactgttg aagagcttca aaacttaact 1620
   acgttgccca gtattcagca atcagtaatc ttgaacgatg tggtaaatac tactgacaat 1680
   ccagatactg ttttgggtat cagaatgact ttcgataatc agaacttgaa gactggttac 1740
   aatgtgcata ctgttggtat ttatgcaaaa gaaccgggga aaaatgaaat tctttacggt 1800
   atagctacgg ctaagacacc agaatatatt csagatttta gcgagcaaac tttatttaag 1860
   tttgatttct taatgtatct agtaattggt agaacagata aggtaactgt agaagttagt 1920
   cctgatgatg tttaccgtaa garagataca tataataaga ctgaaattga tgctaaagtt 1980
   tcagatgccg tatctaagct aaataataaa gatgctgaaa tccttaatag cttgaatgat 2040
   tataagsaag aaaataagac ttatcatcaa aatctatgaa caacaaatca gcgataaact 2100
   ttgatgacta aagccgcata agactgctgt cagaacaaca acttggaasa aaagctgata 2160
   aggccaatac ttacaccaga gatgaagtga atagcaaagt cgctcctaaa gctgatcmaa 2220
   ggttatgtag atagtgaact gaataaaaaa gctgataaag cgactactta tactaaagac 2280
   gaagtaaata gcaaaattgc tgggcagatt aagtcagtaa atgggcacac tgctaatgct 2340
   agtggtgcag taaccttgcc aactttgaaa gctaatacgc ttaccggcta tgatgtaaag 2400
   aacaagacag ctacttttga caacaacgct cattttgatg caaatggcat cttttcaaga 2460
   tggcctgtag atcaaggcgt tatcgggcaa cttgctgatg caattaatgc gaacgtacca 2520
   attgaagaag gacctgccaa tactgaccta ttaatgaatg caggtaataa aatagtttat 2580
   tggcatggca atggggatgc agttaagaat ttaccgccaa tgaataataa aaaatatttt 2640
   tttgctgtta agttatttga tactaattgg ggttctgtaa ctgttataga tcaagacgga 2700
   ggttactgga taaatacagc gaatggcaat gtttggactg gttggcgttc agtcgtcaca 2760
   aatgaacatc ttaaaaagtt gaaatttgta aagcagtcat tagatcaaag tggtaatact 2820
   ttccaagata ctaagtttgt aacgcaagaa gcagatggaa catataagat taacattttc 2880
   gatagcgact ggactgctaa taaggttgca tggctattga aaaatactag ttcttataac 2940
   attggtgcta atactaactt aaatgatatt aaagctcctg gtctttatca ttgttccggt 3000
   gcatctaata tagcgaatct tcctgctggc gaggatagct ggtttaatat ggtcgttaac 3060
   agtgataatt ggaatgggtc acaagcttta tatgcaacaa atcataatca actctatatt 3120
   aggacttgga caaatagtgg ttttaaggca tggcgaagaa tagcaaatat agatgactta 3180
   actaaccaaa gcattacttc aattactgat tatgatgtag ctaacgaagg ttggcacaac 3240
   actcaagttg gaaaattcaa tgcagaaggg cactttgcaa actttttagt tgatgcaggt 3300
   gcattaaaac caattgcgca ttcaattaat gttttaaatt ctaacgcatt aatgaaatct 3360
   ggcggtacta tgaacaagta ttgttggatt atttggaacg gtaacggtgc aaatggcagt 3420
   aaagatggac gtattggtgg cttgcaatgg acgggtgcta cagatagtat tcaaatttat 3480
   ggcgataata atgctgctga taatttggat ttagctattc agttgggtga cgataattct 3540
   aaccatatct caatcaggga taaggacgga acagaaagag cagcaattac agccgacggt 3600
   cactttactg gtagtacaga ttgggcacgt gtaggtggaa caggacttaa caaccctact 3660
   ttaggtagat acattcagca tagtaatact tggaacgaca ttatcggtca aaacaatggg 3720
   caaatggctc ttacagctat tcgtgatgat tctaacgggt cagaaaatac aattggtcaa 3780
   cactctgctg gagttgcttt tggtggtgga gatacaaaag gtgtcttaag tgttgcttgg 3840
   gacaaaccac aggcaagaat tattggtggt aatggcaatt cacctacatg gaaaaaagat 3900
   attgcttgga aagatgatat taataatgcc aatcttcggt tacggtaatt aacagttttg 3960
   acgttgctaa tgtgaaagaa ggacctcatc agacttggcc tgtaaaacag ccttggctgg 4020
   tggatcagat ggcattacac ccgtttgcag acgcaatcaa tgcgttgaat actaatttaa 4080
   gtactttaag aacagaatta atgaatttga aacggcgtgt tgactataac tctccgcaag 4140
   gtgaatttaa caacacaacc gtcaatatta atgacttgag gagtactggt atttatcgtt 4200
   ttgcaaattg ttatattcaa aatggaccat atccgactaa taatactcac tggatttatc 4260
   tacaggtgac agtatttgat gctaatacgg tttatcagac gctctatgaa ggagataaca 4320
   tgtatggtag aaagtcatct agtcctacta attggggtaa atggcataaa tatcttaacc 4380
   aagaagttta attagtttag accttagttt atctaaggct tttttattgg agaaaaaatt 4440
   atggaagcag aaaatttttc aaaattattg aacgaagttg taagtaacgg aaaaattaat 4500
   ttttattatt attcagatcc cactagccca aacattgaat tgcatcactt agcaattcct 4560
   ttcccaggcg aattaagtcc agtggattta ccttatcgtt ggcatgcaga aaagccaagt 4620
   gaagatctag ttgacgcagt ttgggacgat gactcgcata gttggattga aaatagtgat 4680
   aaatctcaac cagctttaat tgctaagttg caagcaagta atgcagctat gcaaaagaaa 4740
   atggaaaact acgaagcagc taagatcaaa gatgctcaaa ataatgacaa agttgctcaa 4800
   gctttgagtg gcgtacaaag gggacaagca caaactacag cagttcttgc tcaacttgtg 4860
   ccaatggttc aacaactttc caagtcagta aacacacccg acaagccaaa tgaagctgat 4920
   gaaactaaaa aagaagaagg tgctgaataa tgtttgattt cgatttcagt tctatctatt 4980
   caaaccttga aagtttgtgt aaatcttcat tagataatgg atactttaca gacaacacca 5040
   ttgcaggatt tgttcagcaa gggacatttg atgcagatgg ctataagaga attacgggtg 5100
   atgagtatgt tgcaggaaat caaagcactg tggcaaacgg ctaaacttaa tttacttcat 5160
   ttgattttgg gaagcttgct cacagctttt ggaattgttc ttttagtgaa cgattccttt 5220
   ttttattggc cgccagaatg cagtggttct tcaataacga tttagtagat gcttttgcaa 5280
   tcatagttgg tattggcttg attgcatttg tttttgctgg tggaagaagt caacttgcta 5340
   atgctgtttt attagcctgt tcagcattct ttctgatgat gctgacagtc ttgcaactag 5400
   gacatgtttt tgtagctcat gactatagca gattgctttc aattgtagcg ctaattgggt 5460
   ggctattagt aattcaatat ttagcagtat tttctaagac tgtaaaacga cgaaagtagg 5520
   tgataagaag tgcaagattg ggctaactta atccgtgaaa tagcacttct tttttctggt 5580
   tttgttgcag gacttactgc ttggaatgct ctacgcaaga ctagtcatga ggtttcaaaa 5640
   gacgataaag aagaattgag agctgaccgt gatttatata gaaatcgttg gcttgaaagt 5700
   gagaaagctt ttgatgaaat tgatgcagaa aatgacaagt tgcgcaagaa ggttaaacgg 5760
   ttagaaaaca agatagatga ttttaaagaa aaagaggata gaaaatgagt ttaggacaaa 5820
   ttacagactt ggttattgtt gctctatcgg tagttgtgac aataatcttt tatttttatt 5880
   caaagcacaa aattgaaatt gataagaaag cactgcaagg agacgcatta gctaaggctg 5940
   agcaaatgat tgctagatct gcaggagcaa ttgtttttca aactgacaaa gaaggtggat 6000
   caggccaaga aaagctggta agtgccttta attacttaac tgctatctta gacatggcac 6060
   acttaccgca cccttcgaca gcgtacatca aaggtgagat tgagaagttg attactacta 6120
   tgaaacaggc taagaatgct gttgaaagta ttcaaacagt aactgctcag tataagccag 6180
   ttgaaaagac agaaacaaaa aaggaggaaa aaataatgct taaaatggtt gatgtatata 6240
   gtggaagccc tagaagtttt gcaactcaaa gtggtactga tatcactatg gtaaaagcta 6300
   ctcaaggaac ttactatgta aacccatact gtgataccga ttatcaggct gctaagaaag 6360
   ctggaaaaat gcttggggta tatcactatt tggtggtggc gatcctgctg ttgaagctaa 6420
   ttatttctat aaaaatacca agaattacat tggcgacagg ttcccgcctt agattgggaa 6480
   gattatcaaa atcctaaatt cggtaaagac tcaaattggt gtagagagtt tgttgataag 6540
   taccacgaat tatctggtgt ttggcctctt atatatactg gtcaagcttc actttctgaa 6600
   gttggaaatt gtgcgaaaga ttgtggtcta tgggtagctt ggtacgctac tatgaattgg 6660
   aacagttgga ctttaccgac tgcaagcttt agtgttgcgc cttggccaac ttataccatc 6720
   tggcagttta cgggtggtga tatggatcgc aacgtggtta acacgacccg tgaaggttgg 6780
   caaaagttag ctaagcctaa tgttgatgga agacctgtat cacaaccagt aattgcagaa 6840
   acaaaactac gtccagaagt taagaaatgg actgatgatc tcggggatgt ttggtactct 6900
   gaaaagggca ctttcgtaac gagcggacga atcaatctta gatatggagc tagaacttca 6960
   tctaaaatta ttgctcaact cccagcaggt acagaagtta agtatgatgc ttatagccga 7020
   cacgatggat atgtctggat tagacaacct cgatcaaatg gatacggata cttagtatgt 7080
   cgtgctggta atgaggcatg gggaactttt aaataattga atacatagga aaagccactc 7140
   tggagttaat tctctggagt ggcttttttt gttgtaaatg tatatacgat ggtatatact 7200
   tacaaatata gagaggatat agtatttaaa aggagcgtgt cgcatggaaa tcgtattaca 7260
   gaaatggggt aatcacaagg gataaggctt tctaaggcta tattaaaaga tyttggaatt 7320
   acttctgaaa aagcaacctt tgaggttgaa ataaaagata aagaattgat tttaaaaaag 7380
   aaaaaagaaa gtaaattagc ccaacgcttt gaaggatttg attacgaagc atactgggaa 7440
   aagtgggaaa gagaacatcc tggtcaatca aaagaagctg actggggaga accagtagga 7500
   aaagaaatta aatggtaaaa atatcactaa tagaaaggag gtgaatatat tggcgataga 7560
   tattaaccaa ggtgatatta ttatcattgg gtttgatcct agtttagggc acgaacaaaa 7620
   aggaaaccgt ccagctttag ttgtatctaa tcatgatttt actagactta cacgatcatt 7680
   agttaaagtt gtgcctatta gtacaacaga caataagttt cctttacata tccctttgcc 7740
   taaaggatta aaagtttatg gagttgctga agttcaacaa gaaacaacgc ttgatctwag 7800
   tgctagaaaa tggaaaaaaa ttgaccatgt ttcagggaac tttttaaaag atgttctagc 7860
   tgcagtcgaa gaatcttatg aacaagagca ataaaaaaag ccactagtgg gaagaactag 7920
   tgacaaggat agagcttata tacttcttag atgttaagat aagtatataa gctttttttg 7980
   gttttgtaaa ctctgacccc caaaatgccc cccaaaaaaa gtaaaacact ataaaagtat 8040
   atagaaaaat aatttttcta aaagtaccac aaaagtacca caattttgat aaatattgat 8100
   ttttttagaa agttagtgaa aactatgaaa gacgttaaat cccttgataa cactaaagaa 8160
   tcgcagtatg acacggctat ctttgctggc ggatgtttct gctgcatggt tgctcctttc 8220
   gatacatatc ctggtgtcat aaaggtcgaa tcabgttata cgggtggggt cgtgcctaac 8280
   cctacttacg aacaagtttg tacgggatta actgggcata ctgaggctgt tcgcattact 8340
   tatgatccaa ataaactatc ttataaggat ttagttaata tttattggca agttactgat 8400
   ccaaccgatg ctatgggaca gtttcaagat cgtggcagtc aatatcaacc agttatttac 8460
   tataattcac ttgaacaaaa agaaattgct gaacaatcac gtaaatcttt agcagattca 8520
   aataagtttt cagatccaat tgtaacccgt attgaaccag caaagccttt ctatttggct 8580
   gaagattacc atcaagattt ctataaaaaa gatccagctc gttatgctct agaagaagcg 8640
   ggagggagag aacaatttat caaagaacat tggaaaaaat agacttgttt ttaactataa 8700
   atatctttac actagatacg ttataaacat agtaaaagag gaaaattatg agtacattag 8760
   aaagsstttc gcgacgccat attttaattt caaagttatc agcagctttg ttttatgctt 8820
   tagccgttss tatcgcctta aactttttct ggcaacctgg taaaatttat gcttctggaa 8880
   ttactggttt tgcccaaatt ttgcagtcgg tatcagaacg atatttgcca tttacattag 8940
   ctacatcagt aatgtatttt gtactaaata tcccattatt tattttagga tggtttaaaa 9000
   ttggccaccg ttttacgatt tttactttaa ttgcagtttt attggcatct attatgatga 9060
   aaattattgc tccaattaag atgagctatg atccaattat ttgtgcaatt tttggtggag 9120
   taattaatgg tgttggtact gggatagctt taaaatcagg tatctctacc ggtggattag 9180
   acgtcttggg aattatctta cgaaagaaaa ctggtaaaag ttttggtcaa ataaatatct 9240
   tttttaacct aattatcgta atctgtgccg gtttkgtatt tggatggact cgagctctat 9300
   atacggcttt aaacatcttt gtcaatggta aggtaataga tgctgtctat aatcaacatc 9360
   aaaagatgca agtcttaatc gtaacagaac atcctaaaca tattattgat ggaattcagg 9420
   ctaaaatgca tcgtgggatt acgattttgc atgatgccga gggtgcatat agtcatactg 9480
   aaaagacagt tttattaatg gtaattgata aatatgatat gtatgacatt tataatattg 9540
   tcttaaataa tgatccgtat gccttcatga gtttaagcga agtttcgaag gtatatggaa 9600
   gattcaaaga acagacgcca gtttagatgt tactagaaaa cgattatttc actttatata 9660
   acaaaggaaa tggtcgtttt tctttaaaaa tatttttata tagggaaatc taacatggaa 9720
   aatcgtttta aaagtgtttt tgatatcatt ggtccagtta tgattggacc ttctagttca 9780
   catacagctg gtgcagtagc aattggcaga gaaggaaaca aactttttgg aggagttcct 9840
   aaaaaggtta ctgttcatta ttatggctct tttgctcaaa ctcatcgtgg acatggtact 9900
   gattatgcaa ttgcagcagg aattttgggc tttactactt ctgacttacg agtaccaaaa 9960
   gccccggaga ttgcccgtaa gcgcggcatt gatattaggt ttgtagagga aaagggagaa 10020
   agcccgattc atcacccaaa tacggcgatt ttagatatga ctaacggtca taaaaaagtt 10080
   caattagcag gctgttccat tggtggtggg gcaatcgaaa ttcgacgcct agtaatccac 10140
   gatattgtgg ttgagccaag cggtacgtta ccaattgttt tattgattga cccagaaaga 10200
   aaaataaaaa atcaaagatc cttaacttcc tttttacaac aaaaggcacc ttttaatgaa 10260
   agcagaattt ataaaagccc tgaatactac atctatgaat atgatattca aaactatttc 10320
   aagctttctt taattggaga actcaagaaa aaatataaaa acattgtttg tttatgaaat 10380
   gagaaactaa tgtacaatca tgttaaagaa attgttgcag acgctgaaaa acaagaaaaa 10440
   cctatttcag aattgataat tgaacaagaa cgcaaattat caggattatc acgagaaaaa 10500
   gtatggaata aaatgaaata taatcttgaa accatgtgtg ctgcggttag taagggaagt 10560
   actggtaaag gagtattttc tcatactggc ttaacgggcg gtgaagcgat taaaataaag 10620
   aattatcgtg aaaaaggtca tactctttct ggtgatttta tgatggaagc tgtgcaaaat 10680
   gccgtagcga caaatgaagt aaatgctgcg atgggcgtca tttgtgcaac cccaacagct 10740
   ggctcttctg gcacacttcc aggtatttta tttatgcttg aaaaaaagct cgacttaaat 10800
   gaagagcaaa tgattcgctt tctctttact gcaggaggaa tgggattagt tattggaaat 10860
   aatgcggaaa tagctggtgc aaccggagga tgccaagctg aagttggttc agcgtccgca 10920
   atgggtgcag cagcagctgt agaaatagct ggtggtacac ctaggcaaag ctcccaagct 10980
   ttagcgattg cgatgtctaa tttattagga ttagtatgtg atccaatagc aggactagta 11040
   gaagtaccct gtgtaaaaag aaatgcaatt ggagctggga acgctctaat tgcagctgat 11100
   atggcattag ctggatgtac tagtgttatc ccagctgatg agtgtattga agcaatgaaa 11160
   aagtaggcca tcaaatgcca gtagcattaa gagaaactgg tattggagga ttagcaggca 11220
   ctccaacagg acaggctatt aaggctagaa tttttggaaa agatgtttaa aatgcgcact 11280
   agtgaagaaa ttaaacaaaa tattattaat caactagcaa ctgtcataga cccggagtta 11340
   ggggtagata ttgttaatct aggactagtt tacgagcttg acttagatga agatggaatt 11400
   tgtctaatta atatgacttt aaccactcca gcttgtcctt taacagaagt attaattgaa 11460
   ttagttaatg ctgcggttat gaaggttcct gaagtcaaaa atgttgatgt tgagttcgtt 11520
   tggtacccag tttggagtcc tgagaaaatg agtgatcagg ctaaaaaaca ttttggatat 11580
   gataaaaaat aatagaagca aaaaaagatc tttcaaaatt taatgaaaga tctttttaaa 11640
   atatttaatt ttatttgtta cttagcgtag tcatcaatac cgaggtgttc taaaattagt 11700
   gctgctgttt cttcaattga acgatgagca acgttaatta catagcaacc aagtttttta 11760
   taaagtttat cagctgcttc aagttctgca ttgatagaat ccatattaga gtaagtagta 11820
   tctgggttca aaccataggc tatcattctt tgtcgcctga tctcgttcaa aacggacgga 11880
   tcatttgtta aaccaatgat tttcttagga ttaatcttat agatttcatc gggaatatgg 11940
   gtttgaggta caagtggtaa attagcgacc ttaagatttt tgttagctaa gaataaagaa 12000
   agtggagtct tagaagttcg agaaacacca agtaaaacaa cgtcagcttc taagaaacct 12060
   tttggatctt ttccgtcatc atacataaca gcaaattcca ttgcagaaat tctatcaaag 12120
   taattttgac ccatgtggtg gacagcacca atttcatgtt taggttcttc gccagttaaa 12180
   gcatgaatgt tatcaattac gccagaaagt aagtcgacgt atttcatatt atgttcacgt 12240
   gcaaatttaa ttacggccaa ttgttcatct tcgtgaatca aggtaaaggc aattaataca 12300
   ttttcaaatt ctgtgatttc actaaaaact ttttctagct tttctcgatt tgtaataaaa 12360
   ggatagcggc ggtagttaaa cttaacattt ggatattgca ccgcaccagc ttgtaccata 12420
   ttgaaagctg tatcaccaac agagtcagaa ataataatta gattaacaat tttttgattt 12480
   tctttttttt cttcagtcat atatttcacc tcattaatta tctaaattat accaaaataa 12540
   gtaagaaaaa tgttaatcga ctattgaccg ttttttctcg tttagctata atttaactat 12600
   attgttgtgg cacatatttg tgtgccaaga aatggaagga gggatctcac atggctaaga 12660
   caatcgttca cgaaaacgag tctattgatg atgctcttcg tcgtttcaaa cgttccgttt 12720
   ctagaagtgg taccttgcaa gaataccgca agcgtgaatt ctacgaaaaa ccaagcgtta 12780
   agagaaagtt aaaatccgaa gctgctcgta agcgtagaca ttattaatat agaaaaagct 12840
   cctggtagaa aattcttcta tcaagagctt tttttatata aaaagtttaa aatagactgt 12900
   gtaagttttg attaaagagg agaattaaat acaatgtcac taaatgatac tttaatgcaa 12960
   gatatgaaaa cagctatgaa agccaaagat aagggagctt taactacaat tcgctcgctt 13020
   aaggcggcag taatgaatta taaaattaaa gtgggacatg atctaactag tgatgatgaa 13080
   ttaacagttt tgtccagcgc tcttaaacaa cgaaaagaat ctttagaaga atttactaag 13140
   gctggcagag atgatttaat taatcaaaca aagaatgaaa tgaaattaat tgaaaaatac 13200
   atgccaaagc aaatgtctaa ggaagaactt gaagaaactg ttgaagaaac aattaaagaa 13260
   gttggagcaa gttctaaaaa agactttggt aaagttatgc aagctttaat gccaaaaatt 13320
   aagggtaagg ctgatggtaa agcagcttct tcaattgttg gtaaaaagct taactaatag 13380
   cttttattaa acggaggatt ttaagtggga caaactacta tagaagcaac atttacacca 13440
   actaaaccag aaacaatcat gaatttagtt ggaattaacg atgcaaattt gcgtttaatt 13500
   gaagaatctt atgatgtgca ggtaactgat actggtagtg aaatcgaagt aaccggagaa 13560
   gaaagcattg ttaagaaaat tatgcaaatt tttactgctt tggataaggt tgttacccgt 13620
   ggagttacta ttactgcaac tgatgttgtt agcgcaatta agatggcaga taagggaact 13680
   ttagaatttt ttggagaatt atataataaa attctaatta gggatgctaa aggtcgtcca 13740
   gttcgcgtta agaatatggg acaaaaaacg ctatattgaa gctattcaaa aatatgatgt 13800
   cgtttttgga attgggccag ctggtacagg aaagactttt ctagccgtag ttatggcgat 13860
   tgcggccttt aaaaaaggag aagtgtcccg gattattttg actagaccag cggtagaagc 13920
   aggcgaatca ttaggatttc ttccagggga tttaaaagaa aaagttgatc catatttaag 13980
   accaatctat gattctttat atgctatttt aggtgtagag ccaaccaacc gtttaatgga 14040
   acgtggggtt attgaagtag ctcctctagc atatatgcgt ggtagaactt tggatgatgc 14100
   ttttgtaatt ctagacgaag cacaaaatac aacacaggca caaatgaaga tgtttttaac 14160
   aagattagga tttaattcta aaatggttgt caatggggac cagacccaaa ttgacttacc 14220
   aggaaaagca aagagtggat tattacaagc tgaacatatt ttacagaata ttgagcaagt 14280
   aaaatttatt aactttactt ttaatgatgt tgttcgtcat cccgttgtgg ccaaaattgt 14340
   tagagcatat gaagaagagg gacactaaga gttgaataat ttagatattt cttttaatga 14400
   tgaagttaat ttcttaaaag atagcgataa agattggatt acatggatta gcaacttatt 14460
   attatcagca aaaaaagaga ttcataaaga gaatactcaa gaaatgagta ttaactttgt 14520
   ttcttccaaa aaaatccatg aaatcaataa aaaataccgc ggcaaagatc gtccaactga 14580
   tgtaatttct tttgcaattg aagatggatt agatgaagac tttattagcg cttttagtga 14640
   cgatcctgat tttgtagaag atattgggga tttatttctt tgccctgaag tcatcaaaag 14700
   acatagtgta gagtatgaaa ctggatttaa tcgggagttt ggatatacac tagttcatgg 14760
   ataccttcac ttaaatggtt atgatcatat cgaagatgat gaagccaagg tgatgtttgg 14820
   tatccaaggt aaagttttac gtgaatatgg acttccgctt catcctgatc aagaaaatca 14880
   cgggaagcaa attcactaaa acttaattaa aaaggatgta acaatgatgg atgaaaagaa 14940
   agactttaaa tcaggttttg tagctttaat tggtagacct aatgttggta aatcaactct 15000
   cttgaattat ttagtggggc aaaaagtggc aattatgtca ccacagccac aaaccactag 15060
   aaataaaatt tcaggtattt atactgatga tcaggagcaa attgttttta ttgatactcc 15120
   tggaattcat aagccaaaaa ataaattaga tgactttatg gataagtcaa gttactctgc 15180
   tttagatgaa gtagatgtgg ttttatttat ggttgaacct gaaccagcag gtaagggaga 15240
   ccaatatatt gctgaacttc ttaagaaaat taaaaagcct gtatttttag tgataaataa 15300
   gattgataaa gtccacccag atgagttatt atctattata gattcatata agaatctagg 15360
   cgattttgct gaaattgttc caatttctgc ttcacaagga aataatgttt ccgagttgat 15420
   taaaacaatt gctaaatatt tgccagaagg tccgcaattt tatgatgcag accaattgac 15480
   tgaccgtcca gaatatttta ttgtagctga attaattcgc gagcaggttt tgaagctcac 15540
   tcatgragaa gttccgcatg c                                           15561
<210> 3
   <211> 11122
   <212> DNA
   <213> Lactobacillus johnsonii
<400> 3
   gtaccatagc catcataaaa ataaaatctg ttagatgctg aataagattt tcttgcattt 60
   gatcagcatc tggtttggcg ataaaaccta tttgtggatt tgtatctgga tcaagctggc 120
   taacatataa tacacgattg tttttaaaat cgaatttagg attaccttca tcatcttctg 180
   gaagcttaaa gccaatcata tacatgtagg cattatcaaa gtattccact tgattagcct 240
   tctgacttat cactttatct aatgcattaa tcagcgtttt aatagaatca aaaattccct 300
   gacgttcttc attttcgaag aactcgactg caggaaccaa tccatatgga tttattgcat 360
   agccggctgc attagtatct tcttcaatat cataaccttt aaatttataa aacttgtcag 420
   catactgaat aactccataa gcatctgtcc aattgttaga attatcaatc tgataatgta 480
   caaatgctaa aggctgacgt tgtactgtat catcataaat aataaaagca tgattagggc 540
   ttgaatacat taagtgcggc cttgcatctt cgccttgata aatactggca atagaacgtc 600
   caaagatatc acactgctta ctaatttcat taatggtatc aaaaaaattt tcttgtctat 660
   tccagcgggc aatttcatca atttttgagc tatcatttaa caaagctagt ttaggctcaa 720
   taccacaaaa gtatccattg tacacatcaa cgacatattt tgctgaatta acgacaatcc 780
   gattatctgc tccagtttcc ttttctggtg cggttaatat cttatgttta cctagatata 840
   acttcatatt ctctctatat ctaggtttca aaactgtttc gttatatgca ataaaaccta 900
   ataattcatt actagttaat ttttctccct taggaaaaat aaagctactg tttccagtaa 960
   ctttatctct tccataatta atatctttca aaatctcact ccctacatga atgatttaag 1020
   tagcgtcgct ttcgtacttc catgaccatt ctcttcctct accgcataac gaatagcatc 1080
   aatacagtgg ttatatgcat cgatcggcct atttgtgtat tcatcggtct tcttatcttt 1140
   cacgtatgtg taattctgca attcttcaat tgtcttaacg caacgatcat caaccaccca 1200
   tttaaattgc tgtagaaatt gaattccttg aattattgaa tcaggacctt ttaatgctgg 1260
   tctaattcga taaataccgt ctcttttcat ttcagcaatt gatttctttt cagcactatc 1320
   agctgttatt acttcttttg aatagcccat atccttaatt acttgtgcta gctgattatt 1380
   aagcaagcct tttttaacaa attcatccat cacataaagt gttttattcc tcatatctag 1440
   cttgatatgc ataaaagcac ttggatcatt gataaatcca taatcaagac caaaataatc 1500
   atttaacgct aataatttag gatcacgagg attaagtcgc ttagtttcaa agtaagggaa 1560
   aacaagttta tctaatgttg caaactcacc taatgtataa atcttgtaat aagctggatt 1620
   agtatttttc agttcttcaa tcgttctaat attgtcttca tctaaaaatc tattatcttt 1680
   gtaagttgat tgatggatag ctactcttga ccgatcataa tctgcactag gatcaaacca 1740
   agtctgataa gtccaattga gttttgatac tgggttaaac atacaaaaaa tttgtcgctg 1800
   tttatgctta ggttcacgca aacgcaaagt aagttgagtg tagtcattat gattaaattc 1860
   acttgcttct tccataacca catcagacag ccccttaatg gatttaatct tttctggatc 1920
   atccatacct tgaaataaaa aaatcgcccc atttggaagg acgattgttt tatcagatct 1980
   atttacatga cagtattgaa ggatattcca tccacttaaa cactcagtta cgtcagtaaa 2040
   aattgaattt ttaactgttc tatcaacctt acgtagccaa agcacttttc ttggaacatt 2100
   ccaatgctgc aatgatttca atacaacttt ttgtactact ccatgacttt taccacttga 2160
   tgcaccgcca taccacactt ctgttaagtg cgaataatca aaaaggttgt taaagatctg 2220
   tttattaaat acttttgagg gatgcggaaa attaagttga atattaacca tcataatcac 2280
   caggatctat gtttactgta atatcggttt gagtttcaac tttatcggta aaaatcttat 2340
   agtatttacc aagtaattct aaagccttta attggtcctg atcttctgga gtaacagtcg 2400
   tgacattatc atattcaact ttaggctttt tccctttata agaaatacgc ttaaaaacct 2460
   tttctctagg ttttccaatt gctaactggc tcaaattatc cagaactcca ttaatatcaa 2520
   gaatatcgtt aactttagtt tgatgcattg tttcgctaag atattcagat acgttatcat 2580
   ttgctatcaa tcttgatcca ttagctctcg cttgattatc actttttact ttataaacag 2640
   ccttatacgc tctagaagca ttgaaatctt ttaaatattc ctgacaaaac ttcttctgtt 2700
   ttggagttag ttctttcaaa ttatcaccac cttaattttt gcaaaataaa aagccagctt 2760
   atgctgactt aaaaatcata aattttccag acaaaactta tattgtttat cgtaatcttc 2820
   aaattcatca attatattaa tactagataa tatctctcca tctttagcta ctaaattaat 2880
   taaaatattt tgaccaccaa tcccaaccct ttctttagtt tttttgtcgt caatattttt 2940
   ttcgattttc attagttctt tgaatatatg aattttatta tttgaatcgc aattaagcaa 3000
   ataatctatc gcttcttcac actgaggctt taatatccaa ttaggatttg aataacttgc 3060
   gatctcctga atatcaaatt tattagtgct cctaagagca taagcatgtg tattcatatc 3120
   ttcatcaaag ccgaagatat aaattgttgt gtctgtattt actttttcga tattattttt 3180
   taaaacatct cgaattatta gactaaatgt tttaacttct ttcgataaaa ttgatctaga 3240
   tctatcaaaa gctagtctaa taattgaaaa atctccagtt ccacacaata tgctttgagt 3300
   aaaaggataa tattcaaatt tttgagtaaa gtaagatgga atagtttttc cattgttgtt 3360
   ttttctcgat gctaaagtat ccatagataa aataatacta ttaggactaa tttcagcatt 3420
   taaagcagtc ataaaatcac ctcaataaca attataaaaa aatcaatttc gaaataatag 3480
   gattcattct ttgagatgaa tataaattta agctaactaa gtccaacaat ggtaaatatt 3540
   ttagaaccac tgctaaaagt ttaataatat ttccattaaa aaagaactgg cctttcgact 3600
   aactcttata taattattta ttcaatttaa acttttctgg attatttttc ataatatcca 3660
   aagccggcgg ttgaagtata tacaaaatcg gaacatcatt tatatcttct atttctccaa 3720
   atttagtata tgatacattc ttagttttaa ctattacttt agattgatat aattcaagaa 3780
   cattaggatc attttcatgt aaatagcctt tatattgagg aggattatta aataatttcc 3840
   taacaatatt tagtttttct tctgtgagac ctaaaacata ttttttatat tcttgctgtt 3900
   cttttttcgt tttatatctt ttccaaatta cgtcgccaat atcaaggcca atcgaaatcc 3960
   aaaaaatcaa tgctacaaaa tatataaaaa gaaaacacat agttaatact ttacccactg 4020
   gcaatgtttc aatataagat cttgacaaca aaactatcgt acaagtaata gctattagta 4080
   atcttgtttt catgtttgac ttaagagcct cccatgctgt ctttactaat tcttccaatt 4140
   aattacctcc attcgtttca aaaataatta taaaaaagcc aagtctaatt aaaagacttg 4200
   actttcttga aacgaacgga attttagtcc taagaaggac tatgggaaat actggaatcg 4260
   aaccaagctt aatgtctcac catttagaga tatttcccac ttgctcttta acaaaataat 4320
   tttgaaggga cattcttaaa gaatgtgtgt atgcgcacgt aacacagagc aatagcctca 4380
   agtgttaaat acataatata gagaggaatt ctattaattc ccatagctcg tacaagaatc 4440
   gaacttgtgt tactggtatg aaaaaccagt gtcttaaacc acttgaccaa cgagccagac 4500
   taggaaagcc gcatctctta tcgtgcgctt ttacgattgt tgcagtgtgt gctttacagt 4560
   ttaatgtagg ggttacatta gtcacatgtt cagttcaccc acctaccgcg aaaacaatca 4620
   acctaataac tgtaaaatta aatgacttat ttcgtgaatt tatcatttaa attactttac 4680
   caattgtatg acaaggtaaa gtttttgttt tacgtccgtt ttccgccgga caatgagcag 4740
   cgtggggatc gaacccactg tatattgtga aagaagattc cttctttcta attccaaatt 4800
   gtgtcatact gcccacgact atcgcgcagt ccactgaacg atagccaact cttaactaca 4860
   atacgctcgc cggggcgtcg catatcagtc ctagatcata gcgtgaccta ggtaagtagc 4920
   aaataggaat cgagcctaaa tgctactgca catcaatcat gagactattt aacagaaatt 4980
   ataaatttaa ccaagatgat aaattaatga ttgatgtgct aggagatctt ttcagatcta 5040
   aaattgtaaa aaatatgaag gattggatct tttaacttga aaaactcata ttaccgtgtc 5100
   ggactttctt actttccgac aatatctatt taagcataga tagttgtcag taaattacca 5160
   ccatattccc acaaaaatac cctatttata tttatgtaaa tcaataatag gctggcaatt 5220
   gttcactctt tgccaatagt caaagccatc agcaaattca aacattgctt tccttcgtaa 5280
   ataactgaac ttgttgtttt cgctataaac taaaggttta acttcattcc atggccattt 5340
   acgtataaat agccctatca aaataatttg agaattatgc gagcaatgat gtattgcatc 5400
   actagtcgct tttaccattg cctcagcatt caatcctcta ataatcgctt tttcactatt 5460
   attacttcca ttactatgag caggagccga acttaatgta ggagacttaa gatctaataa 5520
   ttcatggcca ctcatcaata gcaatcgttc caaatctttg ttaaagaatt ttgctacttc 5580
   ttttagcgtc tctttttcat ccaattctgg aaataaactc actgtatcag ctcctagttc 5640
   tattttctat ccttctaagc cataatctct atatggttca tcatgcgcta agtgatagcg 5700
   cctgtattca ttactcatct atgacatcct taaattcata attcatttcg tctctatcgt 5760
   atttttcata aatcattaaa gctgaaaacg ccagtcctga atcccccata tcccagggag 5820
   taacttgata tttaattttt tctattgtca atggaccttc tttttcaaga aaatcattga 5880
   tggcatcttc taaatattta gatgcacttt cttcaaataa cttaactttc atctttacca 5940
   ccttgtcttt cttaaatcgt atcctgttct agccactttt gtagtccaga agcataatcc 6000
   cttatccagt cactaataac ttttccgtca ggcaagtttt cccaataatc cggatcaaac 6060
   tctacagcta ttttggtttc taaatcagaa ctcatgtgat agcccgttct atcatcccag 6120
   ctttccaaaa tagagttctt taattcttga cgttctgatt ttgataaata gcaatcatca 6180
   ttttcatcgg ataatagtaa ataatcatta agctgttcat ctaaaagact gtaatcataa 6240
   ctccataatt cttctgctgc ttttacttta gaaacaaagt agcctaaatc tttactgtat 6300
   tccgcaatat ctcgcaaagt gttttgactt gaataccagc agaacactgc aaatccaaaa 6360
   tcaccactaa tgcttaacgt tgaacttgac cgatcgaaaa ggtaatttat tcgatttttc 6420
   cattcatatt caggatcttt acaagcaaga tattctattt tgttatcaaa gcatttgtat 6480
   tcaaatacgt ggttattaaa gctcatctta aaactccaac ttaactctct tccgtttatt 6540
   atctgctacc catctagcag ttacttctaa cattaatttt tgaaaatcct cattttgcca 6600
   tgctgactta cctaatagtt gactccgtgc atattcttca ttcaaggtgt ctgcaagctt 6660
   cttgctggtt aatttgctat aggcatcccc tcgactttct tttttctcct ccgcatvaaa 6720
   atcattaaaa gggattggcc aatgctaggt gctttctttt tgcttgatag ccggctgaaa 6780
   ataccccccc cgattaagtt aagagcatct aggtcttcta acggtttatg ctcatcaagt 6840
   ttctttacaa ttgttttatc aacataggcg gtctttagat cacctagtac atcagatgct 6900
   actttcattt aatttctctc cttatatttt ctggctatga agttaatcct tgcagtttct 6960
   agctgcatct ctaagcgtga catagcatat agaactccta aattcaaaag cgcagttatt 7020
   gtacctttag sbattgcacg ttgcgcacct tttctatatt gcttttgcaa ttggttaata 7080
   gtcatttttg cttgtttttt cttcaactta tctctttttc tcatttttaa atccttacac 7140
   aaatctcaaa aagatcaaaa ctatatcaat gattgttaag caagttattg gaacataaga 7200
   cttttgatcg tctctaatta catctataaa aaataaatta attaaaataa aaacgcacca 7260
   ccattcggca gataattgtc cgctcattgt ttttctccaa actctttaaa gattttgttt 7320
   ctttcttcta cagtaatttg cggtgtagaa gattgtttag gacgatatgt actccaatca 7380
   gttgccttac gaacatattt attttgttga ttaaaacgag gcttaatagg tgtcatatct 7440
   aaatcatcat cgaatcgacc attaaaccat gtagatccat taagggtata ttctagtggg 7500
   gtttgtttaa gcttcagaaa acgaawgtat ttttctagtt ttcgttcata acctcaaaag 7560
   tattgtcctt acttctgttt cgccaagctt tatagtgcct taatgcagct ttctttcctt 7620
   gttttttagg atagagtgcc cataactgtt cgaattctga gtcataaatt tgggtcggag 7680
   attcggaaga atcgactata ttatttatac tatccttacc taacctatcc ttacctaacc 7740
   tatgcggtcc atttgacgtc cattggttgt ccactggacg tccattattt tcaattgctt 7800
   taacaccagt cttctttccc gtatctgctc ttgctttagc ttgctgaatt tcaatgtctg 7860
   gtagcatttt gacaagtaag tctcgataga tagagtcaac ttttctatcg gaccgaatgc 7920
   ggttattttc tttccaatca tttatataag taaccagatc agcatttaaa actgttataa 7980
   ggcctttagc tgccagaatt tttaaatcat cttcagtaga gccggtttgt cgcatcacgt 8040
   tatagccttc aaccactcca tcatcgtcgg catgtatgcc taaatgaaaa tataaggcct 8100
   gagttgacgg aggcatttta agaaaacggg cgctctctac tattctttgt gagaacattc 8160
   tacgttgtgc caatcatttc accacctaaa acaatttatc tacttcagga tctttagatt 8220
   ctactttctt atcttcaaat ggatcctttt taacagtttg ccgaatttta tcaattgcag 8280
   cttgtttctc cttagtttct tgttttactg aatgattttg cttaaacatt tcggcaattt 8340
   gatgtacagc ctgtgcatct tgcggtgtat gtggtccttc agccagtctt gtgcatcttt 8400
   aatgccatct ttcgcttctt gataaagatc gtataagtta gcttgtatgc cattgtaata 8460
   gactttatag tcctcaagtt cttgtttagt taagatttta ggttcgtaaa tagtcttttg 8520
   atcttgtgca tcatcgtcat tatctgcagc gatcccaaag gctccactaa gtgaatatct 8580
   ttttgcataa ctgataagac tggcggttgc ttgtgcgtcc caagctttat tattttgaaa 8640
   taccacctta ttcgttttga gcgtaaagcc agaagaatct actaaaattg tttgtacttc 8700
   tacagtatta ttttcagttc taatatcgaa aaaatagcta aatacgacat tgccatcttt 8760
   atctgtaact tttttgatac catccattac tgcggcatca atgtcattta aatcagcata 8820
   attatagtca tagctatagg gtgtaccttg cttggttttt ccagaaacag taactttatg 8880
   cgttttttca ggctgtttaa tattagcctt cacttgtgca taatgcattg cccacttagc 8940
   tctgtcttct tcttttccat agatttccat cttagttcac cctctcatag ctaatacccc 9000
   agtctcgaat gaaattagat agggctgtta gctgatcggt agtaccagta agtttaagtg 9060
   tgacgctatg taccttatca actacttcgc ctgttttggc atcgacatac ttatcgccat 9120
   gttgttcaag cgattctata gctttcttac gattttcttc ttgctgttta gcctgcttaa 9180
   ttaaatattc atggtcatta tccatttgag ttagtacgtc aggtagcgac ttataatcaa 9240
   gcatttgaag atatggacta gcagtcatag caggtttagt gtactcacta gccttagtag 9300
   caataacttg ctcagcttct tttctagcct ttagtctttc agcaattgct ttaaattgtt 9360
   gtctagcttc ctcctcaatt tttccccagc tagctgtctt gttgtcccac ttttcttggt 9420
   aatcaagctc ctgtaaaggt atattgtatt caactgcaat cttacccagt tgcaataaat 9480
   tctgttgatg tttgtctttt ttagctttat catcaaaaga cttaattcct tcactgatgt 9540
   gatcaactgc taccttaatt ttcaaatcaa gtgatttaat ttgagcagta aactcatcaa 9600
   taggttgact agcttgttgt gtaattgatt ttcttcgctc atttaaggct tttcgaatgc 9660
   gatttaattc tgccctagtc tttttatcat aaggatacga tttagaagtt actacatagc 9720
   tttcccatcc tttagctaaa tcatctactt tagcatttag tgactcgtac ccaggaaagt 9780
   ctatttcagc ctttttaaaa ctaattggga aattttcatt ttcaaacgta atcatgtctt 9840
   ttttagcttc gttaatctta ttcatgttat aattcctttg gttttgtatt ttttgtagtc 9900
   gttaaactgt tggcgcagtt aacggctttt ttgttgtcat taaatctgtc tcaaattgtt 9960
   tttgatattc atccggtata agcggttgtc ttgtttctct agtgaagaaa gcattactgt 10020
   tactgattaa ttttgtttct aatgtcattt atctcatctc cctaaatatt tggaaaaatc 10080
   gctgtataca tcgtaaaaat aagtgctact aatgctgtac acatggtacc tagcgtaagt 10140
   atttctgttt cacgtactgt gaaatcagta cccatgaatt cgttgatctt gtggttaatc 10200
   catttactca tagtttgtga tcctccataa acttttgtaa gacatccttg tcatataaga 10260
   tttggccatc aatactaatt ggcttaaaat caaacttttt cagccaaatt ctaaatgtag 10320
   atgggctaac tcctaagaag tttgctgatg ctgtcacgtt aaaatacttc tgttgaatag 10380
   ctctttcaat aactcgttct gggattgcga tttgcatgaa atcatctcct ttttaatgaa 10440
   tcaaaatact ttccttacta gctctatcta ctccttttac agcaagataa gtttcttgct 10500
   gaagatggat aaaacttaga atatcttgaa tttcttctgg tttaccttct agtgtcacct 10560
   tcatttttat ctcctctcaa ttcatcaaga ctaacgccta acgcatcagc aattttgcac 10620
   atagttttga atgtaggctc agtctttaaa tatctataat ttctaattgt ttggtaattt 10680
   attttcgatt catttgataa tttacgtaaa ctccaatgct tattttttaa aatacgatca 10740
   actgtttgcc acgacatata gtgttatcac tttcctatct actttatatt gtgttatatt 10800
   tgatacatga tattttttag aaagaagaga catagtggca actcaatata aacaatctat 10860
   ctgcttaaat ggtcatcaga ttactgatga atttagtccc aacgaaaccg taactggcta 10920
   ttgcgaacaa tgtggcgcta agcttattga tagctgtccc cattgcaaat atcctattga 10980
   aggattttat tatccagacg gagtagtata tttacgaagt ccaaatgata aacttccagt 11040
   acctaaatac tgtaaaaaat gtggcacacc atatccttgg actaaagatt ctttagatgc 11100
   attgaatgaa gtaatacaat tg                                          11122
<210> 4
   <211> 21
   <212> DNA
   <213> Lactobacillus johnsonii
<400> 4
   acgccagctg gcgaaagggg g 21
<210> 5
   <211>24
   <212> DNA
   <213> Lactobacillus johnsonii
<400> 5
   gctcactcat taggcacccc aggc 24

## Description

The present invention relates to a prophage DNA sequence of lactobacillus johnsonii, and its use in determining instability of lactobacilli cultures.

Lactobacilli are used in factories for the production of dairy products such as yogurt, milk drinks, and white cheese. Since the introduction of such strains in applications on industrial scale no substantial phage problems have been reported. Yet, due to the increasing number of applications more and more fermentation failures were notified, which could not be attributed to bacterial contamination.

The problems reported were not observed in initial small fermenter cultures, yet during fermentation on large scale the number of lactobacilli reached a level that constantly dropped during further fermentation to up to 80 to 90 % of the initial cell count. This drop of the viable cell number could not be explained by lytic phage infection which causes no measurable problems at low phage titers but produces a dramatic, greater than 90% loss at high phage titers.

It was initially hypothesized that these problems might be merely due to nutritional conditions prevailing in large scale fermenters, such as e.g. a lack of growth factors in the larger volumes, or due to technical circumstances, e.g. linked to the mechanical treatment of the cells. Also genetic inclinations were envisaged wherein gene expression of the cells were deemed to change under conditions of mass culture wherein a bacterial suicide system could be induced (Lepeuple et al., Appl. Environ. Microbiol. 64 (1998), 4142-4148).

Consequently a problem of the present invention is to find means for determining and/or extinguishing biological factors responsible for instabilities in factory fermentations.

The above problem has been solved by providing the nucleotide sequence of the prophage contained in Lactobacillusjohnsonii Lal CNCM I 1225, identified by SEQ ID No. 3.

These sequences or parts thereof may suitably be used for determining the presence of prophages in Lactobacilli.

According to another preferred embodiment the above sequence may be used to improve the stability of lactobacilli cultures.

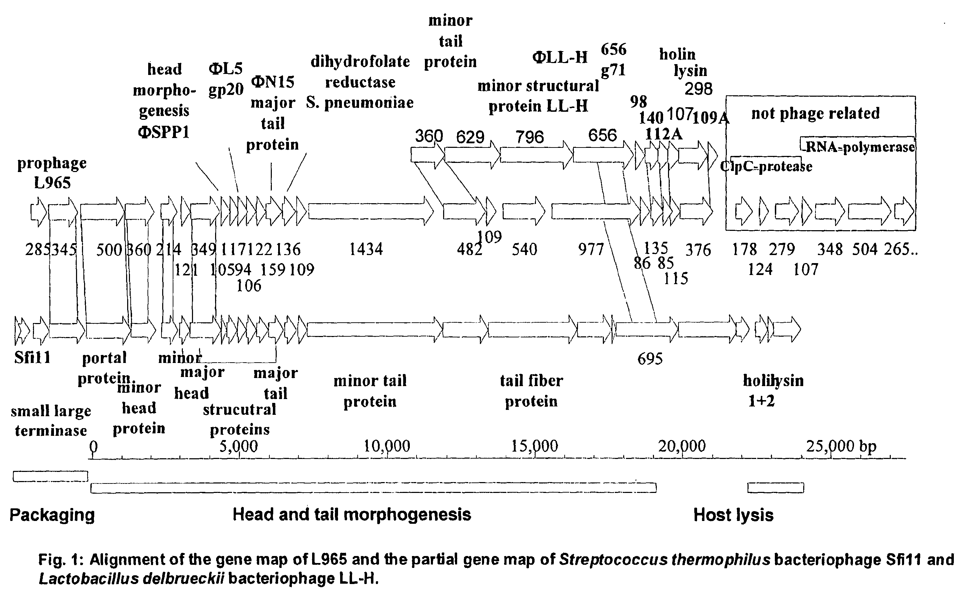

Fig. 1, 2 and 3 show the prediction of open reading frames attributed to phage sequences and their respective polypeptides

Prophages are the genomes of temperate phages that have integrated into the bacterial genome encoding inter alia lytic enzymes to free the phage from the cell after induction. Autolysins are one group of such lytic enzymes, polypeptides that en-zy-ma--tically act on the bacterial cell wall. The liberation of some prophages from the genome may be induced by mutagens, such as DNA damaging agents (e.g. mitomycin C) or UV light and are also activated when the cell experiences other stress situations. Though prophage DNA present in the bacterial genome may be incomplete and is hence in-ca--pable of producing viable phage particles they may still be capable of encoding lytic enzymes.

During the experiments leading to the present invention it was therefore decided to investigate the genome of L. johnsonii for the presence of prophages, in order to exclude the possibility that temperate phages may be responsible for the instability of latobacilli cultures. This screening was not trivial, since no genetic information on the structure of L. johnsonii phages was known.

The La1 genome dataset available from the Genome project was screened with bacterio-phage sequences of known Lactobacillus, Lactococcus, Streptococcus and Bacillus phages using the BLAST algorithm. As first screening probes the following phage sequences were used:
- Bacteriophage LL-H, accession number M96254
   (Vasala et al., Appl. Environ. Microbiol 61 (1995), 4004 - 4011)
- Bacteriophage SPP1 complete nucleotide sequence, X97918
   (Becker et al., J. Mol. Biol. 268 (1997), 822-839)
- B. subtilis DNA (28 kb PBSX/skin element region), Z70177
   (Krogh et al., Microbiology. 142 (1996), 2031-2040)
- Lactobacillus bacteriophage phigle complete genomic DNA, X98106
   (Kodaira et al., Gene 187 (1997), 45-53)

When the La1 genome database was searched with the set of phage sequences described three different contigs (the Lal DNA dataset is organized in segments termed "contigs", which range in size from about 500 bp to about 50 kb) revealed phage-related sequences. These contigs were retrieved from the database and then further analysed. In these 3 contigs of various lengths, different amounts of bacteriophage related sequences were found.

In the contig termed L965 (about 35 kb total length) (SEQ ID No. 1), a 25 kb segment of contiguous phage sequences was identified, which codes for a complete packaging, morphogenesis and lysis module of a pac-site containing temperate Siphoviridae most closely related to S. thermophilus phage Sfi11.

The contig L771 (about 16 kb) (SEQ ID No. 2) contains 8kb of phage related sequences, most closely related to B. subtilis prophage PBSX. In contig L771 also a probable lysis module could be identified.

The contig L928 (11kb) (SEQ ID No. 3) codes for a contiguous phage sequence, which spans from the replication region to the packaging region. The contigs are discussed in detail in the following paragraphs.

### Prophage contig L965

Contig L965 is 35.6 kb long and contains 32 open reading frames. Database homology searches revealed 15 highly significant matches and 3 matches of borderline significance. The results of these searches are summarized in table 1.

**Table 1**

| L965 open reading frames and database similarities. The grey bar indicates the end of phage-related sequences. ORF's are named by the number of aminoacids in the predicted protein, the BLAST P-value and the scores are given, identity is on aa level. | | | |
|---|---|---|---|
| **ORF** | **Similarity** | **BLAST** | **Identity** |
| 89 | - | | |
| 285 | XTMA_BACSU PBSX PHAGE TERMINASE SMALL SUB. Hypoth. protein 8 (xre region) Bacillus subt | 7e-16 | 62/229 (27%) |
| | | 5e-14 | 48/150 (32%) |
| | YQAS_BACSU similar to phage-related terminase small subunit Bacillus subtilis | 2e-08 | 27/49 (55%) |
| | (Z99110) similar to hypoth. Proteins from. | 2e-08 | 27/49(55%) |
| 345 | (U88974) ORF26 S. thermophilus phage 01205 | 5e-16 | 72/251 (28%) |
| | (AE000790) cons. Hyp. Protein Borrelia | 1e-06 | 45/212 (21 %) |
| | (U 19754) BBA31 homolog Borrelia burgdorferi | 4e-06 | 66/306 (21 %) |
| 500 | (AF057033) gp502 S. thermophilus phage Sfi11 (U88974) | 2e-39 | 127/446 |
| | ORF27 S. thermophilus phage 01205 | 2e-38 | (28%) |
| | (Y11901) Lactococcus lactis | 4e-16 | 123/446 |
| | SIZ_BPSPP portal portal vertex protein SPP1 | 1e-08 | (27%) |
| | | | 70/259 (27%) |
| | | | 106/480 |
| | | | (22 %) |
| 360 | gp284 S. thermophilus pahge Sfi11 | 2e-09 | 39/155 (25%) |
| | ORF28 S. thermophilus phage 01205 | 5e-09 | 75/341 (21 %) |
| 214 | (U88974) ORF2 S. thermophilus phage 01205 | 5e-10 | 49/169 (28%) |
| | (AF057033) gp193 S. thermophilus bacteriophage Sfi11 | 2e-09 | 48/168 (28%) |
| 121 | (AF071201) bacteriophage Felix 01 | 3.4 | 16/53 (30%) |
| 349 | (AF057033) gp348 S.thermophilus phage Sfi11 | 6e-24 | 88/311 (28%) |
| | (U88974) ORF31 S. thermophilus phage 01205 | 1e-23 | 87/311 (27%) |
| 105 | (Q38584) product required for head morphogenensis | 0.00014 | 24/98 (24%) |
| | (AF009630) E14 bacteriophage bIL170 | 0.0087 | 11/61 (18%) |
| | (O21901) bacteriophage sk1 | 0.011 | 11/61 (18%) |
| 117 | | No | Similarity |
| 94 | Mycobacteriophage L5 gp20 | 0.30 | |
| 106 | | No | similarity |
| 122 | | | |
| 159 | INVA_YEREN Invasin Yersinia enterocolitica | 3e-05 | 32/153 (20%) |
| | (O64327) gp13 Bacteriophage N15 | 0.0032 | 32/237 (23) |
| 136 | (AF055723) Dihydrofolate reductase Streptococcus pneumoniae | 5e-05 | 33/123 (26%) |
| 109 | | | |
| 1434 | Myosin | | |
| 482 | (AF032121) S. thermophilus phage Sfi21 orf 1560 | 161 1e-38 | 92/216 (42%) |
| | (AF032122) S. thermophilus phage Sfi19 orf 1620 | | 99/262 (37%) |
| | (L02496) phage LL-H orf 999 (X98106) minor capsid protein phage phigle orf1608 | 150 2e-35 | 102/273 (37%) |
| | YQBO_BACSU | 147 1e-34 | 64/167 (38%) |
| | XKDO_BACSU PBSX protein XKDO | | 84/303 (27%) |
| | (AB009866) orf 16 bacteriophage phi PVL | 102 4e-21 | 77/244 (31 %) |
| | (AB009866) orf 17 phage phi PVL | | 0/235 (25 %) |
| | (AF009630) 116 bacteriophage bIL170 | 86 5e-16 | 32/114 (28%) |
| | | 83 4e-15 | 25/96 (26%) |
| | | 53 3e-06 | |
| | | 40 0.026 | |
| | | 32 5.7 | |
| 109 | ? | | |
| 540 | (AF053947) lambda host specific protein J Yersinia pestis | 430.003 | 28/135 (20%) |
| | YHYA_BPH44 hyp. 65 kd prot. Hyaluronidase B30566 S. pyogenes phage H4489A (M 19348)ORF S. pyogenes phage H4489A | 43 0.003 | 28/132 (21 %) |
| 977 | (L02496) minor structural protein gp58 LL-H | 58 2e-07 | 38/118 (32%) |
| | (U42580) Paramecium bursaria Chlorella virus | 51 4e-05 | 160/789 |
| | (AF032121) S. thermophilus phage Sfi21 orf1276 | 39 0.11 | (20%) |
| | (X97918) gene 21 Bacteriophage SPP1 | 41 0.036 | 16/44(36%) |
| | (L48605) minor structural protein Lactococcus phage c2 | 37 0.31 | 65/245 (26%) 17/38 (44%) |
| 86 | - | | |
| 135 | (L02496) orf140 Bacteriophage LL-H | 52 2e-06 | 32/100 (32%) |
| 115 | (L02496) holin Bacteriophage LL-H | 36 0.052 | 27/99 (27%) |
| 376 | (X78410) lysin Lactobacillus bacteriophage phi adh | 367 e-101 | 184/307 |
| | (L02496) muramidase Bacteriophage LL-H | | (59%) |
| | (Z26590) LysA Bacteriophage mv4 | 224 5e-58 | 102/222 |
| | LYCA_BPMV1 endolysin mv1 lysin | | (45%) |
| | | 214 8e-55 137 1e-31 | 70/179 (39%) 70/179 (39%) |
| | | | |
| 178 | CLAB_LYCES ATP-dependent clp protease | 95 2e-19 | |
| | CLPC PORPU, CLPA PEA, CLAA LYCES, | 95 3e-19 | |
| 124 | (U40604) CIpC ATPase Listeria monocytogenes MECB_BACSU neg. reg. Of gen. Comp. CipC B. subtilis | 177 2e-44 | |
| | CLAA_LYCES clp protease ATP-binding sub. | 175 7e-44 | |
| | | 169 5e-42 | |
| 279 | " | " | |
| 107 | " | " | |
| 348 | RPOB_BACSU DNA-directed RNA polymerase | 448 e-125 | |
| | RPOB_STAAU DNA-directed RNA polymerase | 426 e-118 | |
| 504 | " | " | |
| 265 | " | " | |

| | | | |
|---|---|---|---|
| BLAST scores and identities in italics are derived by a PAM 250 "substitution matrix", which assigns a score for aligning any possible pair of residues. The default substitution matrix in BLAST is ususally BLOSUM62 | | | |

16 matches were found with known proteins from bacteriophages. These matches were clustered in the left 80% of the contig. The similarities were found to phages from Streptococcus, Bacillus and Lactobacillus. Five predicted adjacent ORF's (open reading frame) showed similarity with S. thermophilus phage fSfi11 and 01205. Two further non-adjacent genes showed similarity with S. thermophilus phages fSfi11 and fSfi21, respectively (Fig. 1). Further, four adjacent genes showed similarity with genes from L. delbrueckii subsp. lactis bacteriophage fLL-H and Lactobacillus gasseri fadh, respectively. One gene shows significant similarity to the cryptic Bacillus subtilis prophage PBSX. Three genes located in the right 20% of the contig showed significant similarity to bacterial proteins.

Six contiguous genes from Johnsonii La-1 showed not only a similar topological arrangement to corresponding genes from S. thermophilus fSfi11 (Fig. 1), but also (except for one predicted protein) highly significant sequence similarities at the amino acid (aa) level. This observation indicates a close evolutionary relationship between phages infecting evolutionary related bacterial hosts as has previously been deduced from the comparative analysis of other genome segments from S. thermophilus phages (tail module, lysogeny modules).
Over the next ten genes a very similar topological arrangement was also seen between both phages: A very characteristic pattern of 8 relatively small genes followed by a very large gene was found in both the L. johnsonii La-1 genome sequence and in the Sfi11 phage sequences, with a striking similarity in ORF length (Fig. 1). However, no sequence similarity was observed between both groups of genes. Previously a similar genomic organization in phage lambda and phages from low GC gram-positive bacteria was observed (Chandry et al., Mol. Microbiol. 26, (1997) 49-64). This parallel allowed the tentative prediction of gene functions based on detailed knowledge of phage lambda. Several of these predictions have been verified in the analysis of the S. thermo-philus phage genomes. According to this new phage bioinformatic concept which is based on the hypothesis of distant evolutionary relationships between Siphoviridae (tailed phages) from many bacterial genera, the following functions for the newly described L. johnsonii La-1 ORF's could be predicted (Table 1). In four cases the tentative attribution of function was supported by bioinformatic links when using a sensitive PAM 250 scoring matrix (see below). According to the lambda model, one would predict ORF 159 to encode the major tail protein of prophage L965 from L. johnsonii La-1.

**Table 2**

| Prediction of gene functions for the La-1 ORF's by comparison with phage lambda gene map | | | | |
|---|---|---|---|---|
| **La-1 ORF** | **Lambda gene** | **Lambda ORF** | **Function** | **BI link** |
| 285 | Nul | 181 | Terminase small subunit | yes |
| 345 | A | 641 | Terminase large subunit | |
| ? | W | | Head-tail joining | |
| 500 | B | 533 | Portal protein | yes |
| 360 | C | 439 | Capsid component | yes |
| 214 | Nu3 | 201 | Scaffold protein | yes |
| 121 | D | 110 | Head-DNA stabilisation | |
| 349 | E | 341 | Major head protein | yes |
| 105 | W | 68 | Head-tail joining | (yes) |
| 117 | FI | 117 | Terminase-vertex binding | |
| 94 | FII | 117 | Head-tail joining | |
| 106 | Z | 192 | Links DNA ends to tail | |
| 122 | U | 131 | Head-tail joining | |
| 159 | V | 256 | Major tail protein | (yes) |
| 136 | G | 140 | Initiator formation, injection | |
| 109 | T | 144 | Tail component | |
| 1434 | H | 853 | Tail ruler, injection | (yes) |

| | | | | |
|---|---|---|---|---|
| The first column gives the length of the La-1 prophage genes in aa. The genes are noted as they follow on the genetic map. The second column gives the corresponding lambda genes when the two phage genomes were aligned. For optimal alignment the W gene is quoted in the topological order found in lambdoid phage P21. The third column gives the aa length of the corresponding lambda genes. The fourth column gives the biological function of the corresponding lambda proteins. The fifth column indicates whether a bioinformatic link from database searches confirmed the tentative gene attribution. | | | | |

Taken together, the leftmost 17 kb of contig L965 comprising 18 ORF's (ORF 285 to ORF 482) showed clear topological similarity to the genomic organisation of a number of Siphoviridae from various bacterial hosts. Sequence similarity over this genome segment was mainly with predicted proteins from S. thermophilus phages. An interesting aspect was the link with proteins from both cos-site and pac-site S. thermophilus phages. The head genes of L965 prophage showed similarity to pac-site of S. thermophilus phages such as fSfi11 or fO1205 while a tail gene (ORF 482) showed sequence similarity with cos-site phages (table 1).

In S. thermophilus phages the large minor tail protein gene is followed by four (cos-site phages) to six genes (pac-site phages) before reaching the lysis cassette. In L. johnsonii La-1 prophage L965 six genes were found between the analogue of the minor tail protein and the lysis cassette.

ORF 115 gp showed 27 % aa identity to the holin from L. delbrueckii phage LL-H. Both proteins showed a almost identical predicted transmembrane profile.

ORF 376 gp showed 59% aa identity with the lysin from Lactobacillus gasseri phage adh over its entire length (P=10-101) and 45 % with L. delbrueckii phages LL-H over the N-terminal 220 aa (P=10-58). It should be noted that the lysin of LL-H is about 80 aa shorter than the L965 lysin. Significant similarity with numerous other phage lysins (Lb. delbrueckii phages mv4, mv1; S. pneumoniae phages CP-1; CP-7, CP-9; L. lactis phages Tuc2009, LC-3; TP21; all P values < 10-8) and an autolytic lysozyme from Clostridium acetobutylicum (ORF 324; P=10-5) were detected.

Notably, the ORF 977 to ORF 376 region of prophage L965 was similarly organised as the lysis module and its preceding genes in L. delbrueckii phage LL-H (Fig. 1). In addition to the similar topological organisation of the ORF's, three non-adjacent genes predicted proteins with sequence similarity to the corresponding LL-H proteins.

The L965 prophage encodes a holin and a lysin protein which resemble closely proteins from Lactobacillus phages. The phage lysins normally lack secretion signals. This was also the case in the lysins predicted for the two La-1 prophages. The phage lysins are thus unable to reach the target of their enzymatic action: the cell wall of the gram-positive bacterium. Export of the phage lysin is mediated by holins (Krogh et al., J. Bacteriol. 180 (1998), 2110-2117). The holins are pore-forming proteins which allow the export of intracellular proteins. There are different forms of holins.

Class I holins could be identified, which span the cytoplasmic membrane three times. Expression of holins is lethal to the cell, even in the absence of lysin expression.

ORF 376 was followed by a relatively long non-coding region of about 800 bp and then by two genes of bacterial host origin encoding ClpA (P=10-19) and ClpC proteins (P=10-44) of the clp protease system, two unattributed genes and a gene encoding a DNA-directed RNA polymerase (P=10-125).

### Prophage contig L771

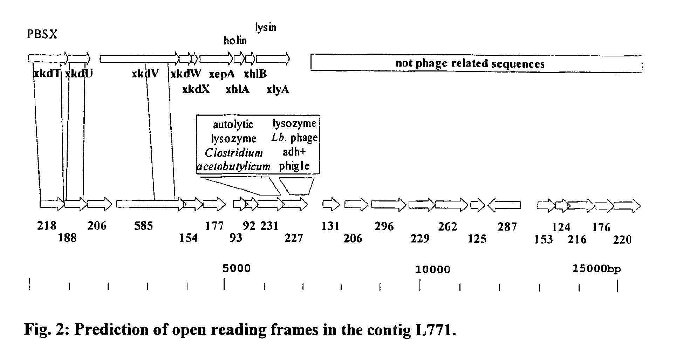

Contig L771 consists of 15.5 kb of sequence information. A tentative gene map (Fig. 2) was established and phage related sequences were found in the left part of contig L771 (0-7 kb), while from 7 kb onwards unequivocal bacterial genes were identified (ORF 131 gp: PemK, P=10-9; ORF 206 gp: peptide methionine sulfoxide reductase P=10-51). The first part showed topologically a very similar organisation to the late gene region of the B. subtilis prophage PBSX. This region comprises a number of unattributed genes (xkdT to xkdX) followed by a lysis cassette. The similarity was not limited to the topological arrangement of the genes, as several genes from the L771 prophage sequence predicted also proteins which showed sequence similarity with the corresponding PBSX prophage proteins.

Clear bioinformatic links were established for the genes in this region. The matches allowed the association of these genes with the corresponding PBSX genes. ORF 231 gp showed 37 % identity over a 60 aa segment of an autolytic lysozyme of Clostridium acetobutylicum (P=10-4). ORF 227 gp showed over a 75 aa segment high similarity to lysins of the amidase class from a number of bacteriophages (Lactobacillus phage adh, gle; Tp-21; all P values < 10-10). The highest similarity was with phage adh (75% identity, P=10-31). This highly conserved phage lysin segment was also found in the putative lysin from prophage L965 of L. johnsonii La-1, notably a 330 bp segment of the lysin genes from both L. johnsonii La-1 prophages shared 70 % bp identity.

According to their position upstream of lysin genes ORF 93 and ORF 92 clearly identify themselves as holin genes. Two arguments are in favour of a holin attribution to ORF 93: class I holin genes encode proteins of about 100 aa length and these proteins show three transmembrane segments.

### Contig L928

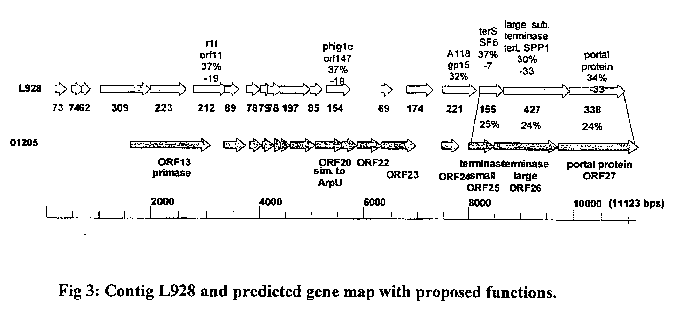

Contig L928 contains 11 kb of sequence data. The sequence predicts 19 proteins (Fig. 3) which show similarities to S. thermophilus, B. subtilis and Lactobacillus bacterio-phages.

The right 7 kb show some similarity to the topological organization of the pac-site region of S. thermophilus phage Sfi11, but the sequence similarity was only significant for the putative portal protein. In addition the potential small and large subunits of the terminase were identified.

The sequence information provided may now be utilized for determining the presence of related sequences in lactobacilli, also other than lactobacilli johnsonii. To this end, a part of the sequence of one of the contigs disclosed, preferably sequences comprising ORF's for holins or autolysins, may be used as probes in hybridisation or experiments using various conditions of stringeny. Moreover, the technique of polymerase chain reaction may well be used for this purpose.

Likewise, the sequence information may be used for extinguishing such sequences detrimental to the growth of lactobacilli. In particular, the genes coding for autolysins and/or holins, respectively, may be deactivated or deleted from the genome of the lactobacillus of interest making use of homologous recombination. Hence flanking regions of each of the contigs or of the respective genes to be deleted/inactivated may be used for effecting homologous recombination, by introducing a DNA sequence containing a deactivated gene or a marker gene in between the respective "flanking sequences" into the bacteria's genome. The DNA segment will then insert at the location of homologous sequences in the chromosome and delete the endogeneous sequences. To this end the use of a marker gene may be suitable to select clones, in which the endogeneous contig's or DNA-sequences have been replaced by the recombinant one.

The sequences or part thereof, in particular short oligoncleotides may be used for monitoring the presence or loss of any prophages in Lactic acid bacteria by DNA/DNA hybridization or PCR reaction. Likewise, the DNA sequence provided by the present invention may be used for monitoring prophage gene expression under different growth conditions, such that the optimal fermentation and growth conditions for the lactic acid bacteria may be selected.

The following examples illustrate the invention without limiting it thereto.

### Examples

### Fragment cloning and clone-bank construction

One mg samples of L. johnsonii Lal chromosomal DNA was digested by 6 base pair sequence recognition restriction enzymes in the search of digestions producing predominantly large, i.e. 8 kb and larger DNA fragments. Seven restriction enzymes identified in this way, BamHI, SacI, SphI, BsrGI, NheI, KpnI and MluI were selected for cloning. 0.3 mg of chromosomal DNA was digested to completion with each of the 7 restriction enzymes and ligated with 0.1 mg of the appropriately prepared pUC19 (Yanisch-Perron et al., Gene 33 (1985), 103-119) (contains a unique MluI site, RDP unpublished results). These ligations were electro-transformed into the E. coli strain BZ234 and directly plated onto LB plates supplemented with 100 mg/ml ampicillin (Boehringer Mannheim, product number 835 242), Xgal (5-bromo-4-chloro-3-indolyl-b-D-galactopyranoside, Boehringer Mannheim, product number 1 680 293) and IPTG (isopropyl-b-D-thio-galacto-side, Boehringer Mannheim, product number 1 411 446). The plates were incubated at 37°C for 16 h and the insert containing colonies were identified by the blue-white color reaction, i.e. white colonies containing inserts. White colonies were picked into 96-well microtiter plates containing 150 ml LB medium supplemented with 100 mg/ml ampicillin and incubated at 37°C to produce mini cultures.

From these microtiter plates, 20 ml aliquots were removed from each of the 12 wells in a given row and pooled in 8 individual Eppendorf tubes to give the 'pools of rows' (i.e. A1-12 etc). From these 'pools of rows', 40 ml aliquots were taken into fresh Eppendorf tubes to give the 'pools of plates'. Finally, glycerol was added to the microtiter plates plus the pools in the Eppendorf tubes and after mixing, frozen at -20°C.

Eight microtiter plates were inoculated with colonies from each of the 7 ligations giving more than 5000 individual clones.

### Clone-bank testing

The quality of the clone-banks in the microtiter plates was tested by restriction analysis of isolated plasmids to estimate the size of the inserted L. johnsonii Lal fragments and the level of repeated fragments. Thirty ml of the microtiter culture were inoculated into 3 ml of LB medium supplemented with 100 mg/ml ampicillin and incubated at 37°C with agitation. Small scale plasmid isolations were prepared using the Genomed mini prep kit (product number 200200) as described in the instructions leaflet and finally suspended in 50 ml TE buffer. Up to 100 plasmids from each of the 7 clone-banks were prepared, digested with the restriction enzymes EcoRI plus HindIII and the fragments resolved on a 1% agarose gel. These restriction enzymes cut at the two extremities of the cloning arrays of the pUC plasmids and thus release the vector from the Lal cloned insert that may be further digested if these sites are present within the insert DNA. This produces a plasmid digestion pattern, or a sort of code-bar pattern, that is specific in the number and sizes of the DNA fragments cloned from L. johnsonii Lal. The direct comparison of these patterns can rapidly eliminate clones that are represented more than once in the clone-bank, regardless of the orientation of the inserted DNA. The sizes of the Lal EcoRI plus HindIII restriction fragments may also be summed to produce the total size of that Lal insert. From these data we have a conservative estimate for an average 4-5 kb insert size, with few clones below this size and with many clones reaching 10 kb and larger. We estimate that each of the 7 clone-banks contains approximately 2 times the Lal genome complement.

The plasmids also showed an impressive physical stability and a high variation in copy number that depended on the nature of the cloned insert. While pUC19 and pUC21 are capable of replication at up to 750 copies/bacterium (and most clones showed this high copy number) some clones displayed a greatly reduced copy number that reflected an E. coli related toxicity of the cloned DNA. These reduced copy number plasmids varied to a point where they were no longer visible on the EcoRI plus HindIII test digestions, with a copy number estimated at 50 copies/bacterium or less.

### DNA sequencing

Sequencing of the L. johnsonii Lal cloned inserts was routinely achieved using the Thermo Sequenase fluorescent labelled primer cycle sequencing kit with 7-deaza-dGTP (Amersham, product number RPN 2538) and the IRD-800 labelled pUC forward and reverse primers. Primer/template mix contained 4 ml of standard template and 4ml primer at 1 pMol/ml in 25 ml volume. Add 6 ml primer/template mix to each of 4 tubes of 200 ml capacity in strip form, plus 2 ml of the sequencing mix, i.e. A to the A reaction etc. The tubes were sealed, mixed and the sequencing took place in an Omni thermocycler using the following conditions; 5 min at 95°C, followed by 25 cycles of 95°C for 30 sec, 50°C for 30 sec, 72°C for 1 min and finally held at room temperature. Three ml of stop solution were added and the samples were frozen at -20°C.

A sample volume of 0.8 ml was loaded onto the Licor DNA sequencer model L4000 with 66 cm plates and 0.25 mm gel thickness. Electrophoresis and sequence reading was by the Licor sequencer. Sequences were downloaded to the GCG suite of programs for sequence compilation and analysis.

### PCR screening of clone banks

The structure of the 7 clone-banks with their resulting pools was designed to facilitate the rapid screening of the clones by PCR. A known DNA sequence taken from the end of a sequenced clone was used to design a pair of PCR primers to produce a PCR amplification product of between 400 and 600 base pairs. A 1 ml sample from the 'pools of plates' was mixed with the PCR primers in a 50 ml volume in the 8 x 200 ml strips with dNTPs, enzyme buffer and 1.25 units of SuperTaq. The reaction volume was heated to 95°C for 5 min in a Perkin-Elmer 9700 PCR machine, then amplified for 30 cycles of 95°C 1 for min, 40°C 1 for min, 72°C for 2 min and finally held at 4°C. 10 ml of gel loading dye was added, mixed and loaded onto a 1.5% agarose gel and electrophoresed at 100 mA for 1 - 2 hrs. A positive control with 200 ng of L. johnsonii Lal chromosomal DNA also used for a PCR reaction and as a size marker for the amplification product and a control of the PCR reaction. The presence of a single positive clone in the mixture of 96 clones in the microtiter plate may be directly seen by the presence of a PCR product the same size as that of L. johnsonii Lal. This technique allows the rapid screening of all the clone-banks in less than 5 hr without the use of radioactivity. Once a positive clone is identified, the PCR detection is repeated on the 8 "pools of rows" for that microtiter plate, and finally on the 12 wells in the identified row.

In case the plasmids are of too low a copy number to be sequenced directly, the plasmids are used as templates for long-range PCR with the appropriate primer from the detection reaction and either oligonucleotide 6617 (5'ACGCCAGCTGGCGAAAGGGGG3') (SEQ ID No. 4) or 6618 (5'GCTCACTCATTAGGCACCCCAGGC3') (SEQ ID No. 5) which amplifies from the pUC 19 vector towards the insert. The PCR product was then gel purified, the DNA eluted and sequenced directly as described for the plasmids.

### Sequence analysis

The Genetics Computer Group sequence analysis package (University of Wisconsin) was used to analyze the sequences. Nucleotide (nt) and predicted amino acid (aa) sequences were compared to those in the databases (GenBank, Release 105; EMBL (Abridged), Release 53; PIR-Protein, Release 55; SWISS-PROT, Release 35; PROSITE, Release 14.0) using FastA (Pearson and Lipman, 1988) and BLAST (Altschul et al., 1990) programs.

Open reading frames (ORF) have been predicted using Clonemanager version 5.0 using ATG and GTG as possible start codons and a minimum size of 90 aa.

### Mitomycin C induction test

The test was adapted from Arber et al., Lambda II (Hendrix, R.W., Ed.) Cold Spring Harbour Laboratory (1983), 443-445. 0.1 ml of a fresh culture was inoculated into a tube containing 10 ml M17 medium supplemented with 0.5 ml of lactose (10% w/v). The culture was subsequently incubated for 1 h at 40° C. Mitomycin C was then added to the final concentration of 0.1 µg per ml. After 6 hours of further incubation the culture was examined by testing the optical density and a plaque assay.

### PCR

The culture supernatant was treated with DNAse to destroy any contaminating, naked chromosomal DNA from lysed bacteria prior to the extraction of the phage DNA (the phage DNA is protected by the phage proteins). PCR was done with primers specific for the L965 prophage.

## Claims

1. DNA molecule consisting of the nucleic acid sequence set forth in SEQ ID No. 3.

2. Use of the DNA-sequence according to claim 1 for determining the presence of prophages in lactic acid bacteria.

3. The use of claim2 to monitor the presence or loss of any prophages in lactic acid bacteria, preferably lactobacilli.

4. The use of claim 2 to monitor prophage gene-expression in lactic acid bacteria.

5. The use of claim 2 for determining optimal fermentation and growth conditions of lactic acid bacteria.

## Patentansprüche

1. DNA-Molekül, welches aus der in SEQ ID NO. 3 gezeigten Nukleinsäure-Sequenz besteht.

2. Verwendung der DNA-Sequenz nach Anspruch 1 zur Bestimmung der Anwesenheit von Prophagen in Milchsäurebakterien.

3. Verwendung nach Anspruch 2 zur Überwachung der Anwesenheit oder des Verlustes von Prophagen in Milchsäurebakterien, vorzugsweise Lactobazillen.

4. Verwendung nach Anspruch 2 zur Überwachung der Prophagen-Genexpression in Milchsäurebakterien.

5. Verwendung nach Anspruch 2 zur Bestimmung der optimalen Fermentations- und Wachstums-Bedingungen von Milchsäurebakterien.

## Revendications

1. Molécule d'ADN constituée de la séquence d'acide nucléique présentée dans SEQ ID N° 3.

2. Utilisation de la séquence d'ADN selon la revendication 1, pour déterminer la présence de prophages dans des bactéries lactiques.

3. Utilisation selon la revendication 2, pour contrôler la présence ou la perte de tous prophages dans des bactéries lactiques, de préférence des lactobacilles.

4. Utilisation selon la revendication 2, pour contrôler l'expression génique de prophages dans des bactéries lactiques.

5. Utilisation selon la revendication 2, pour déterminer les conditions optimales de fermentation et de croissance de bactéries lactiques.
